(19) Europäisches Patentamt
European Patent Office
Office européen des brevets

(11) EP 1 678 498 B1

(12) **FASCICULE DE BREVET EUROPEEN**

(45) Date de publication et mention de la délivrance du brevet:
**18.03.2009 Bulletin 2009/12**

(21) Numéro de dépôt: **04805279.9**

(22) Date de dépôt: **22.10.2004**

(51) Int Cl.:
***G01N 33/50*** *(2006.01)* ***C12G 1/00*** *(2006.01)*

(86) Numéro de dépôt international:
**PCT/FR2004/002716**

(87) Numéro de publication internationale:
**WO 2005/040835 (06.05.2005 Gazette 2005/18)**

(54) **PROCEDE DE DETERMINATION DU POTENTIEL DE DEFENSE ANTIRADICALAIRE ET UTILISATION NOTAMMENT EN THERAPEUTIQUE PREVENTIVE HUMAINE ET VETERINAIRE**

VERFAHREN ZUR BESTIMMUNG DES ANTIRADIKALISCHEN ABWEHRPOTENTIALS UND VERWENDUNG DAVON, INSBESONDERE BEI VORBEUGENDEN THERAPIEN IN DER TIER- UND HUMANMEDIZIN

METHOD FOR DETERMINING THE ANTIRADICAL DEFENSE POTENTIAL AND USE THEREOF, IN PARTICULAR IN VETERINARY AND HUMAN PREVENTIVE THERAPEUTICS

(84) Etats contractants désignés:
**AT BE BG CH CY CZ DE DK EE ES FI FR GB GR HU IE IT LI LU MC NL PL PT RO SE SI SK TR**

(30) Priorité: **22.10.2003 FR 0312351**

(43) Date de publication de la demande:
**12.07.2006 Bulletin 2006/28**

(73) Titulaire: **Prost, Michel**
**21560 Couternon (FR)**

(72) Inventeur: **Prost, Michel**
**21560 Couternon (FR)**

(74) Mandataire: **Clisci, Serge et al**
**S.A. FEDIT-LORIOT & AUTRES**
**38, avenue Hoche**
**75008 Paris (FR)**

(56) Documents cités:
**EP-B- 0 418 335** **WO-A-97/39632**

- **STAGSTED J. ET AL.: "Large differences in erythrocyte stability between species reflect different antioxidative defense mechanisms." FREE RAD. RS., vol. 36, no. 7, 2002, pages 779-789, XP009030764 cité dans la demande**
- **STOCKER P. ET AL.: "ESR study of a biological assay on whole blood : antioxidant efficiency of various vitamins." BIOCHIM. BIOPHYS. ACTA, vol. 1621, 7 avril 2003 (2003-04-07), pages 1-8, XP004416823**
- **LESGARDS J.-F. ET AL.: "Assessment of lifestyle effects on the overall antioxidant capacity of healthy subjects." ENVIRON. HEALTH PERSPECT., vol. 110, no. 5, mai 2002 (2002-05), pages 479-487, XP002280397 cité dans la demande**
- **CHALAS J. ET AL.: "Effect of ethylesterification of phenolic acids on low-density lipoprotein oxidation." BIOMED. PHARMACOTHER., vol. 55, 2001, pages 54-60, XP002280398**
- **KUMAGAI J. ET AL.: "Test for antioxidant ability by scavenging long-lived mutagenic radicals in mammalian cells and by blood test with intentional radicals : an application of gallic acid." RADIAT. PHYS. CHEM., vol. 66, janvier 2003 (2003-01), pages 17-25, XP004399563**
- **YEH C.-T. ET AL.: "Effects of phenolic acids on human phenolsulfotransferases in relation to their antioxidant activity." J. AGRIC. FOOD CHEM., vol. 51, no. 5, 26 février 2003 (2003-02-26), pages 1474-1479, XP002280399**
- **TURNBULL S. ET AL.: "Quinacrine acts as an antioxidant and reduces the toxicity of the prion peptide PrP106-126." NEUROREPORT, vol. 14, no. 13, 15 septembre 2003 (2003-09-15), pages 1743-1745, XP009046190**
- **BARRET A. ET AL.: "Evaluation of quinacrine treatment for prion diseases." J. VIROL., vol. 77, no. 15, août 2003 (2003-08), pages 8462-8469, XP002342471**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Remarques:

Le dossier contient des informations techniques présentées postérieurement au dépôt de la demande et ne figurant pas dans le présent fascicule.

## Description

La présente invention concerne l'évaluation des défenses antiradicalaires d'un organisme vivant. Plus précisément elle vise un procédé de détermination du potentiel de défense antiradicalaire d'un organisme vivant ou de la variation dudit potentiel antiradicalaire induite par un moyen physique (tel que notamment les rayons X, β, γ et UV), chimique ou biochimique chez un organisme vivant, d'une part, et l'utilisation de ce procédé notamment en thérapeutique préventive humaine et vétérinaire, d'autre part.

### *Art antérieur*

Il est rappelé que les radicaux libres sont des substances oxydantes très réactives, qui ont un rôle important dans les phénomènes aigus (tels que traumatisme et ischémie) et qui sont impliquées dans de nombreuses pathologies chroniques.

Dans les conditions biologiques normales, l'organisme vivant produit *in vivo* des radicaux libres et fournit naturellement les moyens nécessaires pour s'en protéger, principalement des systèmes de détoxification enzymatiques et/ou chimiques ; globalement il y a *in situ* un équilibre entre lesdits radicaux libres produits et lesdits systèmes de détoxification.

Cependant des agressions (de diverses natures, notamment les rayonnements UV, le stress, la pollution, l'alcoolisme, le tabagisme, etc.) accroissent la formation *in vivo* de radicaux libres, ce qui conduit à des états pathologiques. En effet, bien que l'organisme vivant soit naturellement protégé contre les radicaux libres par lesdits systèmes de détoxification, ces derniers peuvent être débordés, la rupture de l'équilibre (i.e. le débordement) étant directement liée à des facteurs génétiques, à l'environnement, au mode de vie, etc., bref à la capacité de défense antiradicalaire dudit organisme vivant.

Il est également rappelé que les radicaux libres exercent leurs effets nuisibles :

au niveau cellulaire : peroxydation des acides gras polyinsaturés des membranes phospholipidiques d'où formation de peroxydes cyto-toxiques entraînant phénomènes inflammatoires et mort cellulaire ; de plus, les intermédiaires radicalaires oxygénés représentent de véritables activateurs de la carcinogenèse et des maladies cardiovasculaires notamment ; et,

au niveau extracellulaire : dégradation des principaux constituants d'où modification de la perméabilité et de la structure des tissus, notamment de la peau ; ces altérations contribuent à favoriser la sénescence cutanée et le vieillissement en général.

Les facteurs de protection vis-à-vis des radicaux libres sont essentiellement de deux catégories :

enzymatiques : la superoxyde dismutase (SOD), la catalase et la sélénium glutathion peroxydase ; et,

chimiques : les "scavengers" (i.e. capteurs) qui bloquent les radicaux libres, d'une part, et les anti-oxydants (vitamine C, vitamine A, vitamine E, vitamine K, Se, cystéine, méthionine, ubiquinone ou coenzyme Q) qui limitent les effets des radicaux libres, d'autre part.

On connaît du brevet européen délivré EP-B-0418335 un procédé d'évaluation de l'activité anti-oxydante (i.e. antiradicalaire) d'un agent chimique ou physique à tester, au moyen de la lyse d'un matériau cellulaire, notamment des hématies, par des radicaux libres.

Selon ce procédé :

(1°) on met en contact, dans un milieu biologique liquide approprié, un générateur de radicaux libres et un matériau cellulaire (I) choisi parmi l'ensemble constitué par

- (a) les cellules humaines, animales et végétales,
- (b) les fragments desdites cellules, et
- (c) les parois synthétiques contenant des liposomes,

ledit matériau cellulaire, qui comporte un colorant libérable lors de la lyse par les radicaux libres, ayant été préalablement mis en contact avec un agent chimique ou physique (II) ;

(2°) on induit la libération des radicaux libres à partir dudit générateur de radicaux libres ; et,

(3°) on évalue la lyse du matériau cellulaire par une mesure de densité optique, par rapport à un échantillon témoin contenant ledit matériau cellulaire qui n'a pas été mis en contact avec ledit agent chimique ou physique.

L'un des modes préconisés pour l'évaluation de la lyse à l'étape (3°) consiste à déterminer le temps (T½) correspondant à la lyse de 50 % du matériau cellulaire, en particulier les cellules dudit matériau, et de préférence des hématies (voir à cet effet EP-B-0418335, page 4 lignes 52-55).

Ce procédé connu a été standardisé en utilisant du sang total, humain ou animal, qui est dilué, au lieu des hématies précitées isolées du sang total. Le procédé standardisé a été commercialisé avec succès sous la nomenclature de "Test KRL" (kit radicaux libres) pour déterminer notamment (i) la capacité de défense antiradicalaire d'un tissu cellulaire humain, animal ou végétal, ou (ii) les éventuelles propriétés antiradicalaires d'un agent physique, chimique ou biologique. Plus précisément, le test KRL est réalisé par 1°) mise en contact, dans un milieu tampon approprié, (a) du sang total témoin préalablement dilué, avec (b) un agent à tester (notamment un matériau cellulaire, une substance ou une composition) et (c) un générateur de radicaux libres, 2°) induction de la libération des radicaux libres, 3°) suivi de la cinétique de la lyse des hématies du sang total témoin au moyen de la mesure de l'absorbance du milieu réactionnel, puis 4°) comparaison de cette dernière avec celle du sang total témoin déterminée dans les mêmes conditions sans l'agent à tester.

En variante, on peut avec ce procédé standardisé évaluer le potentiel antiradicalaire d'un sang total d'un sujet humain ou, respectivement, animal par rapport à un sang total témoin de la même variété.

Selon le Test KRL on observe qu'il existe chez l'homme ou l'animal sain, une valeur moyenne de T½ pour exprimer la capacité de défense antiradicalaire. L'article récent de Jean-François LESGARDS et al., publié en mai 2002 dans Environmental Health Perspectives, 110 (No 5), pages 1 à 9, confirme que cette valeur moyenne constitue ou s'inscrit dans une plage moyenne. En dessous de cette plage, le sujet humain, animal ou végétal testé est réputé être dans un état pathologique ou prépathologique en raison d'une insuffisance de défense antiradicalaire.

Malgré l'efficacité du Test KRL on a constaté des anomalies sur sang total dans certains cas particuliers : alors que l'on s'attendait à des valeurs de T½ inférieures à la normale (i.e. inférieures à la plage normale moyenne, comme cela est le cas sur les hématies), on pouvait obtenir des valeurs soit identiques soit supérieures à ladite normale.

Ainsi, chez les patients diabétiques, la valeur T½ évaluée sur sang total est en général analogue ou supérieure à celle des sujets sains, et chez l'oignon fraîchement irradié, la résistance antiradicalaire est supérieure à celle de l'oignon non irradié. Ce sont ces anomalies, que l'on a voulu étudier, qui sont à l'origine de l'invention.

Par ailleurs, de l'article de Jan STAGSTED et al., Free Radical Research, 2002, 36 (No 7), pages 779-789, on connaît le suivi de la lyse d'hématies (isolées du sang total de l'homme, de la vache, du porc, du poulet, du faisan, du rat ou du chat) induite par un agent oxydant, à savoir $H_2O_2$ ou des radicaux libres [provenant d'un générateur de radicaux libres qui est le dichlorhydrate de 2,2'-azobis(2-amidinopropane)] au moyen de la mesure de l'activité de lactate déhydrogénase (LDH) ou de la teneur en hémoglobine du surnageant. Plus précisément, on suit ici la diminution de l'activité LDH ou, respectivement, la diminution de la teneur en hémoglobine. Cependant cet article, qui confirme les résultats, non publiés du Demandeur, obtenus notamment chez les chevaux, bovins, porcs et canards, ne décrit ni ne suggère la séquence des opérations selon l'invention, à savoir : hydrolyse, libération des radicaux libres et évaluation de la lyse induite par lesdits radicaux libres, pour déterminer la résistance antiradicalaire globale.

On sait de l'article de Stuart TURNBULL et al., Neuroreport 2003; 14 (No. 13), pages 106-126 et de l'article de A. BARRET et al., J. Virol., 2003; 77 (No. 15), pages 8462-8469 que la quinacridine est utile en tant qu'agent antioxydant dans le traitement des maladies à prions. Cependant ces deux articles ne décrivent ni ne suggèrent les substances antioxydantes et antiradicalaires de l'invention.

### *Origine et but de l'invention*

On est parti de l'hypothèse que l'organisme vivant comprend : d'une part, des réserves antiradicalaires immédiatement disponibles, c'est-à-dire qu'il existe un potentiel antiradicalaire qui est directement mobilisé contre un choc radicalaire ou stress oxydatif, et d'autre part, des réserves antiradicalaires mobilisables (mais non directement accessibles de façon extemporanée) et qui sont normalement stockées par l'organisme ou bloquées dans l'organisme, le potentiel antiradicalaire global dudit organisme vivant étant représenté par la somme des réserves antiradicalaires immédiatement disponibles et des réserves antiradicalaires mobilisables qui sont stockées.

Selon l'invention, l'on se propose de fournir un procédé permettant de déterminer le potentiel de défense antiradicalaire global en vue de remédier aux anomalies précitées.

Selon l'invention, l'on se propose de fournir également une nouvelle solution technique pour résoudre le problème du dépistage, de la prévention ou de la surveillance des pathologies ou prépathologies liées à une résistance insuffisante aux radicaux libres de l'homme, des animaux (notamment les animaux à sang chaud) et des plantes.

En effet, il existe un besoin (a) chez l'homme ou l'animal, de prévenir l'apparition de pathologies irréversibles telles que les maladies neurologiques dégénératives ; (b) chez le bétail, d'écarter (au plus tard au niveau de l'abattoir) les animaux susceptibles de constituer un risque pour l'alimentation humaine et d'apprécier le stress du transport ou du mode d'élevage ; et (c) chez les végétaux, de suivre la croissance puis le mode de conservation en vue d'optimiser la valeur nutritive des plantes.

Un des buts de l'invention est de chercher à établir une normalité (ou plage de normalité) de défense antiradicalaire par espèce. Un autre but est de pouvoir évaluer un système de régulation des défenses par espèce.

***Objet de l'invention***

L'hypothèse visée ci-dessus s'est révélée exacte, comme démontré et illustré ci-après. En conséquence, on a pu pallier aux anomalies de l'art antérieur et atteindre les buts recherchés.

Selon un premier aspect de l'invention, on préconise un procédé pour la détermination *in vitro* du potentiel de défense antiradicalaire global d'un organisme vivant ou d'un agent physique, chimique ou biologique, ledit procédé, qui comporte l'utilisation de radicaux libres en tant que moyen induisant la lyse d'un matériau cellulaire, qui comporte un colorant libérable lors de la lyse par les radicaux libres, puis l'évaluation de ladite lyse cellulaire, étant caractérisé en ce qu'il comprend l'hydrolyse d'un matériau cellulaire avant ou pendant la libération, dans le milieu réactionnel résultant, de radicaux libres à partir d'un générateur de radicaux libres, ledit matériau cellulaire qui comporte un dit colorant étant des hématies ou du sang total.

On recommande en particulier un procédé pour la détermination *in vitro* du potentiel de défense antiradicalaire global d'un organisme vivant ou d'un agent physique, chimique ou biologique, ledit procédé, qui comporte l'utilisation de radicaux libres en tant que moyen induisant la lyse cellulaire, étant caractérisé en ce qu'il comprend les étapes suivantes :

(α) hydrolyse d'un échantillon

(i) d'un premier matériau cellulaire d'un organisme vivant, ledit premier matériau cellulaire étant un tissu cellulaire, qui est du sang total, ou des cellules, qui sont des hématies, ou, respectivement,
(ii) d'un second matériau cellulaire associé à un agent physique, chimique ou biologique, ledit second matériau cellulaire étant un produit de référence qui est un tissu cellulaire, qui est du sang total, ou des cellules, qui sont des hématies,

(β) mise en contact, pendant ou après ladite hydrolyse, dudit échantillon avec un générateur de radicaux libres,
(γ) induction de la libération des radicaux libres à partir dudit générateur de radicaux libres, et
(δ) suivi de la lyse, par mesure optique, du premier ou, respectivement, du second matériau cellulaire, par rapport à un échantillon témoin, pour apprécier le potentiel antiradicalaire global dudit organisme vivant ou, respectivement, dudit agent physique, chimique ou biologique à tester.

La mesure optique est ici réalisée au moyen d'un spectromètre. On mesure avantageusement la densité ou l' absorbance du milieu d'essai.

En variante, pour apprécier la résistance antiradicalaire d'un agent physique, chimique ou biologique, on peut remplacer ledit second matériau cellulaire par un colorant dégradable par les radicaux libres. Le procédé correspondant est caractérisé en ce qu'il comprend les étapes suivantes :

(α) hydrolyse d'un échantillon
d'un colorant dégradable par les radicaux libres et qui est associé à un agent physique, chimique ou biologique,
(β) mise en contact, pendant ou après ladite hydrolyse, dudit échantillon avec un générateur de radicaux libres,
(γ) induction de la libération des radicaux libres à partir dudit générateur de radicaux libres, et
(δ) suivi de la lyse, par mesure optique, dudit colorant par rapport à un échantillon témoin, pour apprécier le potentiel antiradicalaire global dudit échantillon contenant ledit agent physique, chimique ou biologique à tester.

Selon un second aspect de l'invention, on préconise l'utilisation de ce procédé pour dépister, prévenir ou surveiller *in vitro* les pathologies et états prépathologiques de l'organisme vivant liés à un potentiel de défense antiradicalaire anormal.

Dans cette optique, on préconise une telle utilisation vis-à-vis des maladies neurologiques dégénératives, notamment :

- l'encéphalite spongiforme, qu'il s'agisse de l'encéphalite spongiforme bovine (BSE ou maladie de la vache folle), de l'encéphalite spongiforme ovine (OSE ou tremblante du mouton) ou de l'encéphalite spongiforme humaine (CJD ou maladie de Creutzfeld-Jakob),
- la maladie d'Alzheimer, et
- la maladie de Parkinson.

On préconise enfin une nouvelle utilisation, caractérisée en ce que l'on fait appel à une substance antiradicalaire pour la préparation d'un médicament destiné à un usage préventif en thérapeutique humaine ou vétérinaire vis-à-vis de l'encéphalite spongiforme, de la maladie d'Alzheimer et de la maladie de Parkinson.

***Brève description des dessins***

Dans les dessins annexés :

- la figure 1 illustre le mécanisme de la variation de densité optique (DO) ou de l'absorbance par la lyse de cellules, ici des cellules sanguines ;
- la figure 2, illustre schématiquement le mode de détermination de DO½ et des autres paramètres : on mesure la DO initiale (moyenne des cinq premières valeurs) et la DO finale (moyenne des cinq dernières valeurs minimales), on en déduit la valeur DO½, qui est telle que

$$DO½ = 0,5 \text{ x } (\text{DO initiale-DO finale}),$$

on calcule la valeur T½ qui est le temps de DO½ [la valeur T½ est le temps qui correspond à l'intersection de la droite horizontale passant par DO½ avec la courbe DO = f(T)] ; on mesure la vitesse maximale ($V_{max}$) de lyse radicalaire comme étant la pente de la courbe au voisinage de son point d'inflexion (DO½, T½ ), et on détermine le temps de latence (LT, "lag time") comme étant le temps correspondant à l'intersection de la droite DO initiale avec la droite de $V_{max}$ ;
- la figure 3 montre les courbes Absorbance = f(T) de la quercétine et de son homologue glycone, l'isoquercitrine, à des concentrations croissantes (0 à 100 µM) sans l'hydrolyse préalable de l'invention ;
- la figure 4 donne les courbes T½= f (concentration en quercétine ou isoquercitrine) établies sans hydrolyse préalable à partir des courbes de la figure 3 ;
- la figure 5 donne les courbes T½ = f (concentration) de la quercétine et de l'isoquercitrine obtenues avec hydrolyse enzymatique préalable ; la comparaison des courbes de la figure 5 avec celles de la figure 4 illustre l'intérêt de l'hydrolyse préalable selon l'invention ;
- la figure 6 montre l'influence du traitement de vignes au moyen d'un engrais foliaire par rapport aux vignes non traitées ;
- les figures 7a et 7b montrent que, à l'instant T = 0 ($T_0$), la densité optique (DO) de deux colorants dégradables sous l'action de radicaux libres croît de façon linéaire avec la concentration desdits colorants, qu'ils soient ou non associés à un générateur de radicaux libres (GRL) ; en revanche, pour une concentration donnée en colorant, les figures 7c et 7d montrent que la DO varie en fonction de la libération dans le temps des radicaux libres provenant du GRL ; par ailleurs, les figures 7e et 7f montrent la variation de la DO desdits colorants en fonction de la concentration en GRL libérant les radicaux libres ;
- les figures 8a à 8i concernent la résistance antiradicalaire du sang entier, des hématies et du plasma de quatre lignées de gallinacées (A1, A2, B1 et B2), ans libération des réserves (test KRL de l'art antérieur) et avec libération selon l'invention des réserves 1 (R1) par hydrolyse au moyen d'une glucosidase, des réserves 2 (R2) par hydrolyse au moyen d'une sulfatase ou des réserves 3 (R3) par hydrolyse au moyen d'une glucuronidase ;
- les figures 9a à 9d permettent la comparaison des résistances radicalaires de rats témoins et de rats rendus diabétiques (par administration de streptozotocyne), sans hydrolyse (test KRL) et après hydrolyse (évaluation des réserves R1, R2 et R3) ;
- les figures 10a et 10b concernent l'utilisation du procédé selon l'invention pour le suivi du vieillissement du vin.

***Description détaillée de l'invention***

Les anomalies précitées sont expliquées, selon l'invention, par une mobilisation anormale des réserves antiradicalaires de l'organisme vivant. Ainsi chez les diabétiques, (i) la résistance antiradicalaire du sang total, qui est anormalement élevée, traduit une mobilisation anormale au niveau du plasma des réserves antiradicalaires, et (ii) eu égard au stress oxydant développé chez lesdits diabétiques, la résistance antiradicalaire des hématies, qui sont fragilisées par des réactions de glycosylation, est anormalement faible.

Selon l'invention, on considère que les réserves antiradicalaires mobilisables résultent notamment :

- d'une polymérisation de composés antioxydants et/ou
- d'une conjugaison desdits composés antioxydants avec différents sucres (par exemple glucose, rhammose, acide

glucuronique, etc.), alcools et acides organiques ou inorganiques (tels que l'acide sulfurique), et

que lesdites réserves antiradicalaires mobilisables sont stockées dans l'organisme vivant sous forme de polymères (en particulier des polyphénols), d'éthers, d'esters d'acides organiques et/ou de sulfates, notamment au niveau des parois cellulaires et surtout dans le liquide enrobant les cellules.

Lors d'une agression radicalaire, ces réserves antiradicalaires mobilisables, stockées dans l'organisme, ne sont pas directement disponibles. Elles sont libérées selon un mécanisme enzymatique complexe du type "cascade" avec semble-t-il un système d'inhibiteurs et d'activateurs (à l'instar de la cascade de l'hémostase), pour arriver à un équilibre final. Quand l'équilibre atteint n'est pas l'équilibre normal, on est en présence d'un état pathologique ou prépathologique.

Dans le procédé de l'invention on libère par hydrolyse les réserves antiradicalaires mobilisables qui ont été stockées. L'hydrolyse de l'étape ($\alpha$) est conduite dans un milieu approprié, notamment un tampon, à une température comprise entre 15 et 60°C. Elle est mise en oeuvre soit avant l'étape ($\beta$) précitée, soit en même temps que l'étape ($\beta$). Dans ce dernier cas, on gagne du temps mais il est alors nécessaire d'utiliser un témoin car ladite hydrolyse peut ne pas être achevée avant la lyse du premier ou du second matériau cellulaire par les radicaux libres.

Cette hydrolyse est (i) une hydrolyse enzymatique, (ii) une hydrolyse acide ou alcaline, ou (iii) un clivage au moyen d'un agent physique, notamment un rayonnement.

L'hydrolyse enzymatique est avantageusement effectuée pendant au moins 40 minutes (notamment quand ladite hydrolyse se poursuit pendant l'exposition au champ des radicaux libres), de préférence pendant 1-3 h et mieux pendant 2 h, à une température de 20 à 40°C, avec une substance choisie parmi l'ensemble constitué par les osidases (notamment glucosidases, rhamnosidases, glucuronidases, etc.) les déalkylases (notamment déméthylases), les estérases, les sulfatases, et leurs mélanges. Un mélange d'enzymes, qui convient selon l'invention, peut notamment être du type "glucosidase + déméthylase + sulfatase" ou "glucosidase + glucuronidase + sulfatase".

L'hydrolyse acide est avantageusement effectuée pendant au moins 40 minutes (notamment quand l'hydrolyse se poursuit pendant l'exposition au champ de radicaux libres), de préférence pendant 1-3 h et mieux pendant 2 h, à une température de 40 à 60°C, de préférence à 50°C, à un pH inférieur ou égal à 5,5.

L'hydrolyse alcaline, qui n'est pas essentielle mais concerne plutôt le clivage des fonctions ester et lactone, est avantageusement effectuée pendant au moins 40 minutes (notamment quand l'hydrolyse se poursuit pendant l'exposition au champ de radicaux libres), de préférence pendant 1-3 h et mieux pendant 2 h, à une température de 50°C, à un pH supérieur ou égal à 8,5.

L'hydrolyse selon l'invention peut être un clivage par un agent physique tel qu'un rayonnement, par exemple un rayonnement $\gamma$ ou (mieux) $\beta$ analogue à celui utilisé pour la conservation des récoltes agricoles. L'agent physique peut même être constitué par un champ de radicaux libres appliqué pendant une période suffisante. Cette "hydrolyse", qui consiste à cliver les groupes ester (i.e. acyle), éther et sulfate, d'une part, ou à séparer le fragment oside de glycones, d'autre part, peut donc mettre en oeuvre une irradiation de l'organisme vivant ou d'un matériau cellulaire provenant dudit organisme.

Le matériau cellulaire, qui intervient dans le procédé de l'invention, est choisi parmi l'ensemble constitué par :

(a) un tissu cellulaire (i.e. les cellules, qu'elles soient identiques ou différentes, plus le liquide ou le plasma qui les baignent naturellement ou une partie de celui-ci), par exemple le sang total,
(b) les cellules isolées de leur environnement (et remises le cas échéant en suspension dans un tampon de dilution),
(c) un fragment cellulaire (i.e. un fragment de paroi cellulaire avec le cas échéant la totalité ou une partie du matériel contenu dans la cellule par exemple les lipoprotéines), ou
(d) une paroi synthétique contenant des liposomes.

Le premier matériau cellulaire que l'on soumet à l'hydrolyse de l'étape ($\alpha$) est avantageusement choisi parmi les éléments (a) et (b) ci-dessus, les éléments (a) et (b) (i. e. le sang total et les hématies visés plus haut) étant les préférés, les éléments (c) et (d) étant des équivalents de (a) et (b). Ledit premier matériau cellulaire est utilisé pour apprécier, selon l'invention, le potentiel antiradicalaire global d'un organisme vivant.

Le second matériau cellulaire est choisi parmi les éléments (a), (b), (c) et (d) ci-dessus, les éléments (a) et (b) étant également les préférés. Il est utilisé pour apprécier les éventuelles propriétés antiradicalaires d'un agent physique, chimique ou biologique à tester, d'une part, ou le potentiel antiradicalaire global d'un organisme vivant par le biais d'un produit qui en provient (notamment tissu cellulaire, sang total, cellules isolées, plasma), d'autre part.

L'agent physique, chimique ou biologique est dit associé au second matériau cellulaire. Cela signifie qu'il a déjà contaminé l'organisme à partir duquel on recueille le second matériau cellulaire ou qu'il a été mis en contact, pour la mise en oeuvre du présent procédé, avec ledit second matériau cellulaire.

L'agent physique peut être une exposition à la fumée, aux UV ou à un rayonnement dangereux.

L'agent chimique peut être toute substance ou association de produits chimiques que l'on veut tester pour

déterminer l'éventuelle protection qu'il peut conférer vis-à-vis des radicaux libres.

L'agent biologique peut être toute substance ou association de produits biologiques, par exemple une plante, un extrait d'une substance naturelle, le plasma sanguin ou des cellules pouvant être différentes du tissu cellulaire ou des cellules isolées constituant le second matériau cellulaire.

Comme indiqué plus haut, le second matériau cellulaire peut être remplacé par un colorant dégradable par les radicaux libres afin d'étudier la résistance antiradicalaire globale d'un agent physique, chimique ou biologique. Parmi les colorants qui conviennent, on peut citer notamment les colorants d'oxydoréduction. On a obtenu de bons résultats selon l'invention avec le bleu de méthylène (colorant "bleu" utilisé ci-après) et l'extrait d'oligo-polyanthocyanidine (OPC ; colorant "rouge" utilisé ci-après). Le colorant utilisé ne doit pas être sensible à l'hydrolyse à laquelle on soumet l'échantillon qui le contient. Si par exemple un colorant est sensible à la glucuronidase, il sera utilisé selon un processus mettant en oeuvre une hydrolyse au moyen d'une sulfatase.

Comme indiqué dans EP-B-0 418 335 précité, on préfère parmi les générateurs de radicaux libres, ceux qui présentent une cinétique ou vitesse de réaction d'ordre 0 (la libération des radicaux libres est constante dans le temps) et mieux d'ordre 1 (la libération des radicaux libres est linéaire dans le temps). Les générateurs de radicaux libres oxydants préférés selon l'invention, sont, d'une part, le dichlorhydrate de 2,2'-azo-bis(2-amidinopropane) qui donne dans un milieu aqueux une cinétique d'ordre 1, et d'autre part, le 2,2'-azo-bis(2,4-diméthylvaléronitrile) qui donne dans un milieu liquide huileux ou organique également une cinétique d'ordre 1. La libération des radicaux libres à partir d'un générateur de radicaux fibres est réalisée selon une méthode connue en soi, par exemple la chaleur, la lumière (notamment la lumière du spectre visible ou les UV), les protons, les électrons, les rayons X ; de préférence cette libération sera initiée par des photons, ou par la chaleur. Par exemple, le fait de porter le dichlorhydrate de 2,2'-azo-bis(2-amidinopropane) [en abrégé AAPH] en solution aqueuse à la température de 37°C suffit pour déclencher la libération de radicaux libres selon la réaction :

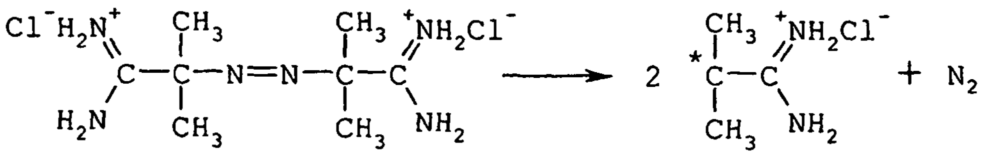

Après le déclenchement de la libération des radicaux libres, le suivi de la lyse du premier ou du second matériau cellulaire est réalisé par voie optique. Plus précisément, on observe la variation de la densité optique (ou de l'absorbance) en fonction du temps, et détermine la valeur T½ (en minutes) qui correspond à la lyse de 50 % du matériau cellulaire. Comme indiqué plus haut ledit matériau cellulaire sera de préférence un tissu cellulaire (sang total par exemple) ou des cellules isolées.

En pratique, la mesure optique est réalisée en automatique, par exemple toutes les 50, 100, 150, 200 et/ou 300 secondes, pour suivre la cinétique de la lyse du premier ou du second matériau cellulaire. Quand on observe la variation de l'absorbance d'un milieu d'essai (contenant par exemple du sang total ou des cellules isolées) au cours du temps à partir de l'instant de l'induction de la libération des radicaux libres du générateur de radicaux libres (en particulier AAPH), on remarque :

- au début une période au cours de laquelle l'absorbance est constante (et relativement élevée), il s'agit de la période de latence au cours de laquelle les cellules n'ont pas été lysées ;
- dès que la lyse cellulaire commence, une phase pendant laquelle l'absorbance décroît de façon linéaire (le point d'inflexion de la courbe correspondante détermine la valeur T½); puis,
- quand toutes les cellules ont été lysées, l'absorbance est constante (et relativement faible).

Ainsi, en opérant sur du sang total, on a selon la technique antérieure du Test KRL, lorsque la valeur T½ croît (voir schéma 1) :

- une plage "1" d'hypoprotection vis-à-vis des radicaux libres, qui correspond à un état pathologique ou prépathologique,
- une plage "N" de valeurs moyennes normales, qui peut contenir des résultats "faux vrais", qui sont anormalement élevés (comme dans le cas des diabétiques) du fait d'une libération anormale des réserves antiradicalaires non compensée par une induction des défenses, et
- une plage "2" d'hyperprotection vis-à-vis des radicaux libres résultant de la libération anormale d'une part importante

ou de la majeure partie des réserves antiradicalaires mobilisables, et qui correspond à un état pathologique ou prépathologique car l'organisme est alors en train d'épuiser lesdites réserves :

***Schéma 1***
**(sang total sans hydrolyse)**

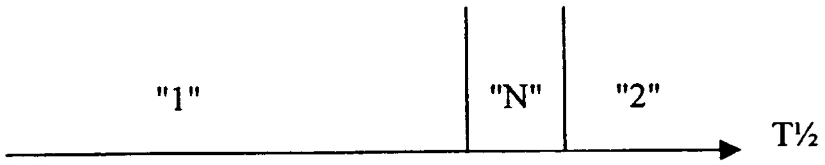


En revanche, selon l'invention, comme on évalue le potentiel antiradicalaire global avec mise en oeuvre de l'hydrolyse, on obtient lorsque T½ croît (voir schéma 2) :

- une plage anormale "A" d'hypoprotection vis-à-vis des radicaux libres, qui correspond à un état pathologique ou prépathologique, ou
- une plage "Nh" de valeurs moyennes normales, qui ne contient que les résultats des sujets sains (une valeur de T½ au delà de ladite plage "Nh" signalerait une erreur de manipulation) :

***Schéma 2***
**(sang total avec hydrolyse)**

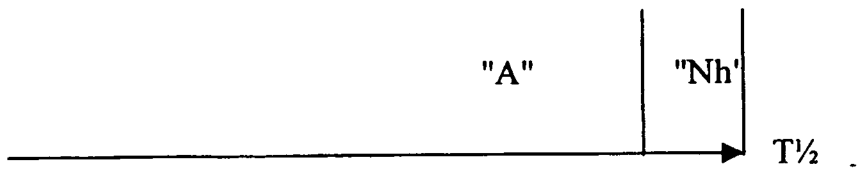


Chez le sportif, par exemple, la plage de normalité Nh est plus large que chez le non-sportif du fait de l'induction des défenses, notamment enzymatiques, stimulée par les efforts répétés ; même si sa défense antiradicalaire diminue ponctuellement lors d'un effort, elle reste régulée dans la plage de valeurs normales contrairement au non-sportif (ou au sujet qui fait du sport occasionnellement), qui lors d'épreuve à l'effort va se retrouver en dehors de la plage de régulation, d'où le risque d'accident cardiaque bien connu des cardiologues.

En conséquence, le procédé de l'invention permet d'éviter tout doute quand la valeur T½ se situe dans la plage "Nh" de valeurs moyennes normales et de supprimer ainsi les anomalies précitées.

Subsidiairement, outre la valeur T½, on peut mesurer, comme indiqué plus haut, le temps de latence, la vitesse de la lyse des cellules par les radicaux libres (i.e. la pente de la courbe) ainsi que l'aire sous la courbe, pour disposer d'éléments complémentaires d'appréciation.

Le potentiel antiradicalaire global peut être également exprimé en unités EAR ("Efficacité Anti-Radicalaire"), 1 unité EAR correspondant au pouvoir antiradicalaire d'une millimole de Trolox® par litre de sang, le Trolox® (Hoffmann-La Roche Inc.) étant un équivalent hydrosoluble de la vitamine E, pris comme antioxydant de référence, ayant pour formule développée :

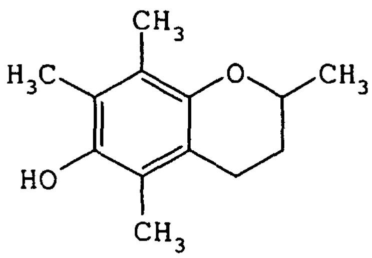

et répondant à la nomenclature systématique de 3,4-dihydro-2,5,7,8-tétraméthyl-2*H*-1-benzopyran-6-ol.

Pour 1 litre de sang d'un homme sain, on a :

1 EAR = $T½_{s\ ou\ h}$ x dilution $_{s\ ou\ h}$ x concentration $_t$ (en mM) x $1/\Delta T½_t$ où "s ou h" se réfèrent au sang total ou, respectivement, aux hématies, et "t" au Trolox.

Pour standardiser le procédé de l'invention, on fait appel à un système, qui est préalablement incubé à la température d'essai (37°C), comprenant :

- une macroplaque comprenant des puits de dilution,
- une microplaque comportant une pluralité de puits d'analyse (notamment 96 puits), et
- un spectromètre destiné à mesurer la DO ou l'absorbance d'une lumière traversant de bas en haut les puits de la microplaque,

Les trois modes opératoires préférés pour la mise en oeuvre du procédé de l'invention sont les suivants :

- mode I, par détermination du potentiel antiradicalaire global sur sang total humain ou animal à sang chaud (notamment mammifère),
- mode II, par détermination du potentiel antiradicalaire global sur hématies humaines ou animales, et
- mode III, par détermination sur plasma.

Par ailleurs en soumettant à l'action de radicaux libres des tissus cérébraux frais de bovins sains, qui étaient dépourvus du prion pathogène PrPsc insoluble, mais contenaient le prion non pathogène PrPc soluble, on a observé l'apparition dans lesdits tissus cérébraux du prion pathogène insoluble. Cette formation de PrPsc a été mise en évidence par utilisation de la technique décrite dans l'article de Sabrina CAPELLARI et al., J. Biol. Chem., 1999 : 274 (No 49), pages 34846-34850, cet article signalant que le PrPc joue un rôle dans l'équilibre redox en prévenant ou contrecarrant les dommages oxydatifs.

On a donc observé de façon inattendue que le tissu cérébral frais de bovin sain produit le prion pathogène de la forme PrPsc sous l'action de radicaux libres provenant d'un générateur de radicaux libres.

De cette observation on a déduit que les maladies neurologiques dégénératives, et notamment BSE, OSE et CJD, sont essentiellement des maladies autoimmunes qui impliquent vraisemblablement deux voies possibles d'infection :

(a) la forme PrPsc est administrée par inadvertance au sujet humain ou animal, et/ou

(b) la forme PrPsc est générée *in vivo* sous l'action de radicaux libres pouvant être produits par l'organisme,

dans les deux cas l'organisme, qui reconnaît la forme PrPsc comme étant étrangère, génère des radicaux libres, via le système immunitaire, pour lutter contre PrPsc. Ce sont ces radicaux libres ainsi générés qui vont transformer la protéine PrPs en protéine isoforme PrPsc pathogène, et produire une agression autoimmune qui explique le phénomène dit de multiplication du prion pathogène.

Un mécanisme identique ou analogue se manifeste (semble-t-il) dans l'apparition des autres maladies neurologiques dégénératives telles que la maladie d'Alzheimer et la maladie de Parkinson. Le système immunitaire attaque les protéines modifiées sous l'action des radicaux libres et reconnues comme étant des ennemis. Ces protéines modifiées étant stables de façon irréversible, le système immunitaire produit des radicaux libres qui attaquent les protéines normales du fait de leurs structures voisines. On a alors une réaction en chaîne.

On préconise donc une nouvelle utilisation en thérapeutique de substances antiradicalaires pour prévenir les maladies neurologiques dégénératives précitées.

Dans cette optique, on fournit une nouvelle utilisation d'une substance antiradicalaire pour la préparation d'un médicament, ladite utilisation étant caractérisée en ce que l'on fait appel à une substance antiradicalaire appartenant à la famille des polyphénols, leurs glycones et leur dérivés sulfatés et notamment choisie parmi l'ensemble constitué par les flavonoïdes et les acides férulique et caféique de formules II et respectivement, III :

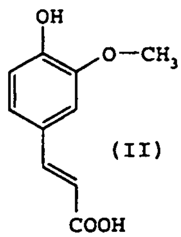
(II)

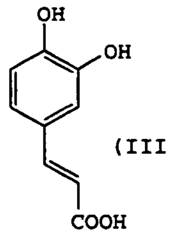
(III)

pour la préparation d'un médicament destiné à un usage en thérapeutique humaine ou vétérinaire vis-à-vis de l'encéphalite spongiforme, notamment l'encéphalite spongiforme bovine (BSE), l'encéphalite spongiforme ovine (OSE) et l'encéphalite spongiforme humaine (CJD).

Dans le cadre de cette nouvelle utilisation, on prévient l'encéphalite spongiforme par administration d'une composition thérapeutique humaine ou vétérinaire comprenant au moins une substance antiradicalaire en association avec un véhicule pharmaceutiquement acceptable.

Ces substances antiradicalaires sont principalement des polyphénols, les glycones qui en dérivent, et leurs dérivés sulfatés. Parmi ces substances, l'on peut notamment mentionner l'acide férulique sulfaté (I). L'hydrolyse de ce produit peut être réalisée selon deux routes distinctes illustrées ci-après par le schéma 3.

Le schéma 3 montre que le clivage par une sulfatase selon 1 conduit à l'acide trans-férulique (II), alors que le clivage par une déméthylase selon 2 conduit *in fine* à l'acide trans-caféique (III), via l'acide trans-caféique sulfaté (IV). L'acide férulique sulfaté (I) présente en conséquence une double réserve antiradicalaire utilisable par hydrolyse. L'activité antiradicalaire de ces produits a été entreprise selon le test ancien KRL précité sans hydrolyse, d'une part, et selon l'invention après hydrolyse, d'autre part. Les résultats obtenus sont consignés dans le tableau I qui illustre l'intérêt de l'hydrolyse selon l'invention.

***Schéma 3***

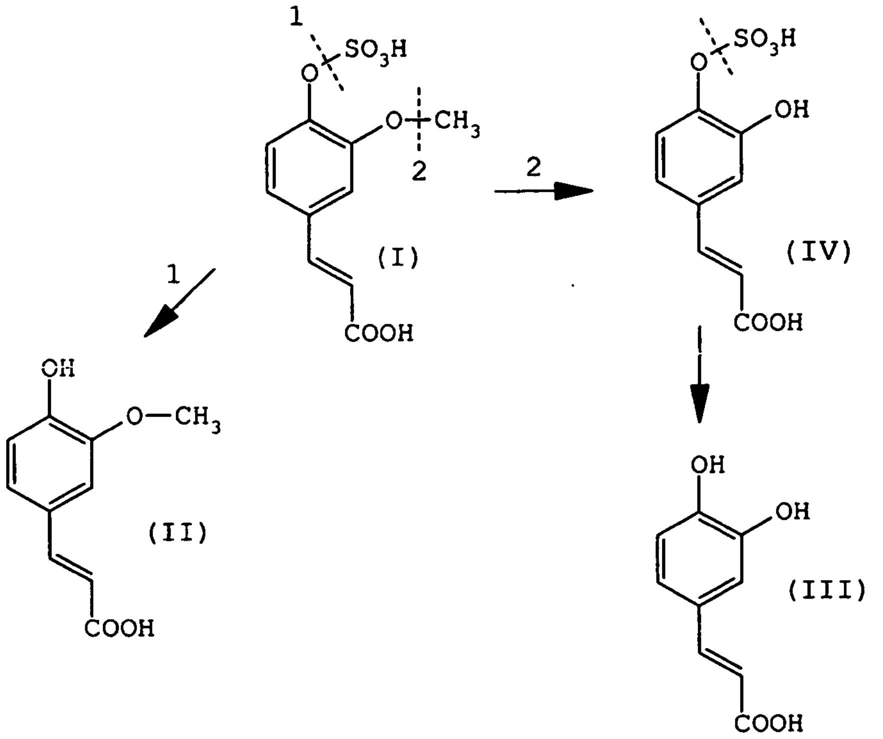


(I) (IV) (II) (III)

***Tableau I***

| Produit | Activité antiradicalaire par rapport à l'acide férulique sans hydrolyse | après hydrolyse |
|---|---|---|
| acide trans-férulique (II) | 1,00 | 2,05 |
| mélange d'isomères cis/trans (47/53 p/p) de l'acide férulique | 0,95 | 1,95 |
| acide trans-caféique (III) | 2,06 | 2,06 |
| acide férulique sulfaté (I) | 0,01 | 2,06 |
| Trolox® (produit de comparaison) | 0,53 | 0,55 |

On obtient des résultats analogues quand on remplace l'acide férulique sulfaté (I) par un oside de l'acide férulique, notamment le glucuronide, et procède à l'hydrolyse avec une osidase appropriée au lieu de la sulfatase.

Conviennent également les polyphénols de la famille des flavonoïdes, les glycones ayant une réserve antiradicalaire plus importante, du fait de la présence d'un ou plusieurs restes osides hydrolysables, que les aglycones, en particulier la cyanidine, la génistéine, la procyanidine, la catéchine et leurs glycones.

Le procédé de l'invention a été standardisé pour opérer avec du sang total, des hématies ou du plasma sanguin en tant que second matériau cellulaire. Dans un réservoir, notamment un puits de macroplaque, on prépare une solution diluée (S) de :

sang (60 μl) dans solution de dilution (1440 μl),
hématies prédiluées à 50 % (60 μl) dans solution de dilution (1440 μl), ou
plasma (60 μl) dans solution de dilution (1440 μl),

on transfère 50 μl de ladite solution (S) dans des puits d'une microplaque contenant une solution de dilution (220 μl), on hydrolyse par voie enzymatique le mélange résultant, on procède le cas échéant à un lavage, on introduit le générateur

de radicaux libres (AAPH) puis on initie la libération des radicaux libres à 37 °C. On détermine ensuite la valeur T½ et les autres paramètres indiqués ci-dessus. Le lavage intermédiaire après hydrolyse peut être omis car il n'est pas essentiel.

L'échantillon à tester (50 µl), qui contient ou est associé à du sang total, à des hématies ou, respectivement, à du plasma et est le cas échéant dilué, est introduit dans la solution de dilution (220 µl) disposée dans des puits d'une microplaque. On hydrolyse, introduit le générateur de radicaux libres, initie la libération des radicaux libres et détermine là valeur T½ et lesdits autres paramètres selon les modalités indiquées plus haut.

De façon pratique, on recommande d'utiliser les quantités suivantes d'enzymes, où U désigne l'unité internationale de l'enzyme concerné :

β-glucosidase à la concentration finale de 11,11 U/ml (3 U/puits), sulfatase à la concentration finale de 3 U/ml (0,8 U/ puits), et/ou
β-glucuronidase à la concentration finale de 2222,22 U/ml (600 U/puits),

D'autres avantages et caractéristiques de l'invention seront mieux compris à la lecture des exemples et essais fournis ci-après. Bien entendu, l'ensemble de ces éléments n'est pas limitatif mais est donné à titre d'illustration.

**Exemple 1** Quercétine / Isoquercitrine

On a apprécié sur un sang total témoin de brebis la résistance à l'attaque radicalaire (au moyen de AAPH précité) en présence d'une forme aglycone de flavonoïde, la quercétine, et de sa forme glycone, l'isoquercitrine, sans l'hydrolyse de l'étape (α), d'une part, et avec ladite hydrolyse, d'autre part.

La figure 3 indique les résultats obtenus avec des doses croissantes de quercétine ou d'isoquercitrine de 20 µM à 100 µM sans hydrolyse préalable.
La figure 4, établie à partir des résultats de la figure 3, montre que la quercétine a un pouvoir antioxydant plus important que celui de l'isoquercitrine quand la valeur T½ est appréciée en fonction de la concentration.
La figure 5 montre que, après hydrolyse au moyen d'un mélange enzymatique β-glucosidase (3 U/puits) + sulfatase (0,8 U/puits) + β-glucuronidase (600 U/puits), la quercétine et l'isoquercitrine ont une résistance antiradicalaire identique. L'isoquercitrine a donc bien été hydrolysée en quercétine par le mélange enzymatique, et la libération de la fonction phénol génère une activité antiradicalaire disponible par rapport à une activité initialement non disponible mais "en réserve".
La figure 5 montre en outre l'activité du mélange d'enzymes [β-glucosidase (3 U/puits) + sulfatase (0,8 U/puits) + β-glucuronidase (600 U/puits)] quand la concentration en isoquercitrine ou quercétine est nulle.

**Exemple 2** Essais sur plasma

Des expérimentations ont été effectuées, selon le mode IIIci-dessus, sur des plasmas congelés de brebis saines (A) ou malades de la tremblante (B), lesdits plasmas intervenant en tant qu'agent biologique en association avec des hématies contrôlées, sans et avec hydrolyse enzymatique préalable au moyen de β-glucosidase aux doses croissantes de 0 U/ml, 1,11 U/ml, 5,55 U/ml et 11,1 U/ml.

Les résultats obtenus sont consignés dans le tableau II ci-après (moyenne de cinq essais par lot, à raison de 3 lots de brebis A, et 3 lots de brebis B ; SD = écart par rapport à la moyenne ; CV = coefficient de variation).

On observe que, sans hydrolyse préalable, les brebis B ont une meilleure résistance antiradicalaire. Il s'agit là d'une anomalie due à une libération des réserves en réponse à l'agression oxydative liée à la pathologie.

Après hydrolyse préalable avec une dose de 1,11 U/ml de β-glucosidase, on constate qu'il n'y a pas de différence notable par rapport aux essais sans hydrolyse.

Après hydrolyse préalable avec une dose de 5,55 U/ml de β-glucosidase, on remarque que le potentiel antiradicalaire global des brebis A est légèrement inférieur à celui des brebis B. Ceci signifie que la réserve antiradicalaire n'a pas encore été totalement libérée par hydrolyse.

En revanche, après hydrolyse préalable avec une dose de 11,1 U/ml de β-glucosidase, on constate que le potentiel antiradicalaire global des brebis A est supérieur à celui des brebis B : la réserve antiradicalaire a été enfin mobilisée par l'hydrolyse.

**TABLEAU II**

| *Essais sur plasma de brebis (dilution 1/135)* | | | | |
|---|---|---|---|---|
| Lots | Vmax (mAU/min) | Lag-Time (min) | T½ (min) | EAR. (eq.mM) |
| sans hydrolyse préalable (glucosidase : 0 U/ml) | | | | |
| B<br>(SD)<br>(CV) | -6,55<br>(0,24)<br>(3,67) | 76,26<br>(4,09)<br>(5,36) | **203,57**<br>(7,32)<br>(3,60) | 35,49<br>(2,45)<br>(6,89) |
| A<br>(SD)<br>(CV) | -7,82<br>(1,26)<br>(16,11) | 77,21<br>(2,50)<br>(3,24) | **186,35**<br>(16,18)<br>(8,68) | 29,73<br>(5,41)<br>(1,18) |
| avec hydrolyse préalable (glucosidase : 1,11 U/ml) | | | | |
| B<br>(SD)<br>(CV) | -6,91<br>(1,22)<br>(17,70) | 98,23<br>(22,30)<br>(22,71) | **216,51**<br>(3,59)<br>(1,66) | 39,81<br>(1,20)<br>(3,02) |
| A<br>(SD)<br>(CV) | -7,33<br>(0,55)<br>(7,47) | 87,11<br>(9,49)<br>(10,90) | **198,48**<br>(11,95)<br>(6,02) | 33,79<br>(3,99)<br>(11,82) |
| avec hydrolyse préalable (glucosidase : 5,55 U/ml) | | | | |
| B<br>(SD)<br>(CV) | -8,06<br>(1,15)<br>(14,28) | 197,49<br>(11,08)<br>(5,61) | **281,37**<br>(1,74)<br>(0,62) | 61,49<br>(0,58)<br>(0,95) |
| A<br>(SD)<br>(CV) | -7,73<br>(0,60)<br>(7,71) | 186,05<br>(13,80)<br>(7,42) | **274,64**<br>(8,92)<br>(3,25) | 59,24)<br>(2,98)<br>(5,03) |
| avec hydrolyse préalable (glucosidase : 11,1 U/ml) | | | | |
| B<br>(SD)<br>(CV) | -9,76<br>(0,56)<br>(5,69) | 260,01<br>(11,91)<br>(4,58) | **318,72**<br>(0,44)<br>(0,14) | 73,97<br>(0,15)<br>(0,20) |
| A<br>(SD)<br>(CV) | -13,00<br>(3,87)<br>(29,74) | 308,91<br>(40,97)<br>(13,26) | **328,84**<br>(13,17)<br>(4,00) | 77,35<br>(4,40)<br>(5,69) |

**Exemple 3** Essais sur vignes

On a utilisé deux vignes, l’une produisant du raisin rouge de table, l’autre du raisin noir également de table. Dans chaque vigne deux lots ont été constitués, le premier recevant un engrais foliaire (contenant des polyphénols) par pulvérisation, le second non traité servant de témoin. Au cours de la croissance, on soumet le broyat de feuilles, le broyat de bourgeons et le jus du fruit exprimé par pression, chacun mis en suspension ou dilution dans un tampon de dilution, au procédé de détermination du potentiel de défense antiradicalaire selon l’invention. On constate que les vignes traitées présentent un potentiel supérieur aux vignes non traitées. Les résultats obtenus avec le jus de raisin sont consignés dans la figure 6.

**Exemple 4** Essais chez le porc

Des essais ont été entrepris sur un lot de 15 porcs, en faisant appel à des animaux réputés sains, l’hydrolyse enzymatique étant mise en oeuvre (pour gagner du temps) pendant l’exposition au champ de radicaux libres libérés à 37 °C à partir du générateur de radicaux libres AAPH précité.

Les résultats obtenus sont consignés dans le tableau III ci-après, où sont fournies les valeurs T½, N’ (plage

de normalité, i.e. plage N en l'absence d'hydrolyse et plages Nh, ici ponctuelles, avec hydrolyse), temps de latence ("lag-time") et vitesse maximale de lyse radicalaire (Vmax), en fonction de l'hydrolyse enzymatique [sulfatase (à 3 U/ml) ou β-glucuronidase (à 2500 U/ml)].

**Exemple 5** Essais chez l'homme

Des essais ont été entrepris sur un groupe de 97 sujets humains réputés sains (hommes et femmes adultes) et un groupe de 92 sujets humains diabétiques (hommes et femmes adultes). Le sang total de chacun des sujets des deux groupes a été soumis (1°) dans une première série d'expériences à une exposition au champ de radicaux libres générés à 37 °C à partir du générateur de radicaux libres AAPH précité, et (2°) dans une seconde série d'expériences à une hydrolyse enzymatique [avec β-glucosidase (3 U/puits), sulfatase (0,8 U/puits), β-glucuronidase (600 U/puits) ou le mélange ternaire précité] mise en oeuvre pendant ladite exposition au même champ de radicaux libres, chaque groupe servant de contrôle par rapport à lui-même.

Les résultats obtenus avec hydrolyse au moyen de β-glucosidase (3 U/puits) sont fournis dans le tableau IV ci-après, qui permet d'observer que chez les sujets sains la valeur moyenne de T½ passe de 92,90 min sans hydrolyse à 263,85 min avec hydrolyse (soit une réserve libérée de 184 %), alors que chez les diabétiques la valeur moyenne de T½ passe de 99,54 min sans hydrolyse à 215,94 min avec hydrolyse (soit une réserve libérée de seulement 116 %). Les résultats obtenus sur plasma humain sont similaires.

**TABLEAU III**

| ***Essais chez le porc*** | | | | |
|---|---|---|---|---|
| Echantillon | T½ (min) | N' (min) | Lag-time (min) | Vmax (mAU/min) |
| sang total | 92,40 | 84-100 | 69,29 | -21,85 |
| sang total + sulfatase | 100,70 | 91-110 | 74,63 | -20,24 |
| sang total + β-glucuronidase | 158,69 | 135-181 | 122,87 | -9,24 |
| hématies | 69,19 | 62-76 | 55,16 | -28,47 |
| hématies + sulfatase | 80,96 | 75-88 | 62,23 | -23,51 |
| hématies + β-glucuronidase | 169,35 | 158-180 | 149,92 | -12,02 |

**TABLEAU IV**

| ***Essais chez l'homme*** | | |
|---|---|---|
| Echantillon | T½ (min) sujets sains sujets | T½(min) diabétiques |
| sang total | 92,90 | 99,54 |
| sang total + β-glucosidase | 263,85 | 215,94 |

L'ensemble de ces essais met en évidence que le procédé de l'invention permet le suivi de la croissance des animaux et des végétaux, en particulier celle des plantes stratégiques telles que les céréales (notamment blé, maïs et riz), les oléagineux (notamment soja, arachide et colza), la vigne et le coton.

Ledit procédé permet également de suivre l'évolution de l'état sanitaire du bétail. Grâce audit procédé on peut déterminer, par espèce, une plage de normalité (i. e. une plage de valeurs normales) en dessous de laquelle il convient de surveiller attentivement les animaux en vue, le cas échéant, d'écarter ou retirer leurs tissus de la consommation. Par application desdits résultats, on recommande plus particulièrement d'administrer aux animaux devant être conduits à l'abattoir un régime complémenté en substances antiradicalaires, notamment l'acide férulique sulfaté, les osides dudit acide férulique et les polyphénols. Un tel régime peut comporter en tant que supplément nutritif un extrait de cacao riche en polyphénols, voire même une composition à base de cortex de cabosse additionnée du mucilage de la cabosse et/ou du jus des fèves de cacao.

L'acide férulique sulfaté, les osides dudit acide férulique (notamment le glucuronide), les polyphénols, ledit extrait de cacao et/ou ladite composition à base de cortex de cabosse sont également utiles chez l'homme pour traiter

et prévenir les états de stress.

**Exemple 6** Utilisation d'un colorant

On a utilisé les colorants bleu (bleu de méthylène) et rouge (extrait d'OPC) précités en vue de standardiser le procédé de l'invention mis en oeuvre sans matériau cellulaire. Les résultats obtenus ci-après sont la moyenne de 6 essais.

Dans une première série d'expériences, on a vérifié que, à l'instant T = 0 ($T_0$) quand on n'induit pas la libération des radicaux libres, la DO (à 260 nm) du colorant bleu est linéaire en fonction de sa concentration, qu'il soit utilisé seul (courbe 1 de la figure 7a) ou en association avec le GRL (courbe 2 de la figure 7a). On constate que la courbe 1 est linéaire:

$$y = 0,09986x + 0,03278 \qquad r^2 = 0,9999$$

et que la courbe 2 est également linéaire :

$$y = 0,0964x + 0,0431 \qquad r^2 = 0,9996$$

Pour le colorant rouge, à l'instant $T_0$, la variation de la DO (à 450 nm) est linéaire, les courbes 1 (sans GRL) et 2 (avec GRL) de la figure 7b étant des droites presque confondues. Pour la courbe 1 on a :

$y = 0,03268x + 0,0608$ $r^2 = 0,9997$
et pour la courbe 2 :
$y = 0,3264x + 0,0511$ $r^2 = 0,9999$

Dans une seconde série d'expériences, on a évalué l'effet du générateur sur chacun des deux colorants au cours du temps. La DO du colorant bleu mesurée à 620 nm est décroissante alors que celle du colorant rouge mesurée à 450 nm est croissante. Voir figures 7c (colorant bleu) et 7d (colorant rouge).

Dans une troisième série d'expériences on a étudié la variation de la DO du colorant bleu (figure 7e), qui est croissante, et celle du colorant rouge (figure 7f) qui est croissante, en fonction de la concentration initiale en GRL à l'instant $T_0$ puis après la libération des radicaux libres.

**Exemple 7** Etude sur quatre lignées de gallinacées

On a étudié la résistance antiradicalaire du sang total, les hématies et du plasma ainsi que la libération des réserves R1 (hydrolyse au moyen de glucosidase), R2 (hydrolyse au moyen de sulfatase) et R3 (hydrolyse au moyen de glucuronidase) sur quatre lignées de poules (A1, A2, B1 et B2) de races différentes : poules de chair (lots A1 et A2) et poules pour reproduction (i.e. poules pour la production d'oeufs ; lots B1 et B2), chaque lot comprenant douze animaux.

Les résultats obtenus, exprimés par la moyenne (Moy) et la déviation standard (SD), sont consignés dans le tableau V ci-après et les figures 8a à 8i.

L'ensemble de ces résultats montre que l'étude des variations des réserves de défense antiradicalaire chez l'animal permet de cibler les critères de performance, comme la croissance, le poids, les performances de la reproduction et la qualité de la production en particulier vis-à-vis des capacités génétiques de résistance au stress et/ou aux maladies.

Tableau V

| Groupes | | Sang total | Hématies | Plasma | Sang total | | | | Hématies | | | |
|---|---|---|---|---|---|---|---|---|---|---|---|---|
| | | T1/2 (min) | T1/2 (min) | T1/2 (a) | R1 (b) | R2 (b) | R3 (b) | Somme (b) | R1 (c) | R2 (c) | R3 (c) | Somme (c)% |
| **A1** | **MOY** | **75,93** | **70,02** | **7,77** | **27,63** | **8,00** | **92,70** | **128,33** | **107,21** | **33,71** | **162,79** | **303,71** |
| | SD | 3,72 | 2,93 | 4,87 | 7,22 | 4,10 | 9,96 | 17,79 | 12,32 | 4,26 | 15,61 | 21,25 |

(suite)

| Groupes | | Sang total | Hématies | Plasma | Sang total | | | | Hématies | | | |
|---|---|---|---|---|---|---|---|---|---|---|---|---|
| | | T1/2 (min) | T1/2 (min) | T1/2 (a) | R1 (b) | R2 (b) | R3 (b) | Somme (b) | R1 (c) | R2 (c) | R3 (c) | Somme (c)% |
| **A2** | **MOY** | **85,08** | **73,73** | **12,81** | **31,29** | **10,55** | **90,55** | **132,39** | **104,62** | **36,11** | **158,00** | **298,72** |
| | SD | 8,71 | 2,98 | 6,03 | 10,93 | 4,69 | 26,38 | 31,47 | 24,97 | 9,41 | 28,28 | 57,44 |
| **B1** | **MOY** | **74,20** | **62,55** | **15,30** | **13,69** | **3,13** | **36,48** | **53,30** | **61,49** | **18,66** | **111,62** | **191,78** |
| | SD | 9,20 | 5,29 | 4,74 | 5,36 | 1,98 | 35,99 | 39,47 | 8,74 | 2,57 | 38,69 | 42,92 |
| **B2** | **MOY** | **79,94** | **69,87** | **12,32** | **21,07** | **3,76** | **56,65** | **81,48** | **98,42** | **19,54** | **144,17** | **262,14** |
| | SD | 8,48 | 6,03 | 4,96 | 6,33 | 2,15 | 25,27 | 30,16 | 25,92 | 2,92 | 28,11 | 40,14 |

Notes
(a) pour le plasma, la valeur T1/2 est exprimée en % par rapport à la valeur T1/2 du sang total
(b) pour R1, R2, R3 et leur somme, la valeur T1/2 du sang total est exprimée en % par rapport à la valeur T1/2 du sang total des témoins
(c) pour R1, R2, R3 et leur somme, la valeur T1/2 des hématies est exprimée en % par rapport à la valeur T1/2 des hématies des témoins

**Exemple 8** Etude sur rats

Des essais ont été entrepris chez le rat normal pris comme témoin et le rat rendu diabétique par traitement à la streptozotocyne. Les résultats obtenus sont consignés dans les figures 9a à 9d.

Les rats rendus diabétiques par la streptozotocyne ont une induction de leurs défenses antiradicalaires plasmatiques libres (défense plasmatique = défense sang total - défense des hématies). Cette induction se fait au détriment des réserves circulantes libérées en particulier pour les réserves R1 qui chutent de plus d'un tiers, la valeur T½ reste pratiquement identique chez les hématies des témoins et des diabétiques puisque nous sommes en début de diabète. On remarque que les réserves R1 et R2 sur les hématies restent presque identiques entre les témoins et les diabétiques et que les réserves R3 intra-cellulaires augmentent pour protéger les hématies au détriment des réserves plasmatiques.

**Exemple 9** Suivi du vieillissement de vins

Les essais ont été effectués sur cinq vins différents, le vin 1 ayant plus de 40 ans, le vin 2 ayant 14 ans de moins que le vin 1, les vins 3, 4 et 5 proviennent de la même parcelle de vignoble pour les années respectives 1992, 1997 et 2000. Le vieillissement est accéléré soit de façon normale en ouvrant chaque bouteille en laissant l'oxydation se développer, soit de façon artificielle au moyen de radicaux libres.

Les résultats obtenus sont consignés dans les figures 10a (test KRL selon l'art antérieur) et 10b (selon le procédé de l'invention). La courbe 10a montre que le vin 2 est totalement madérisé dès lors que son temps de résistance à l'oxydation est trop faible. La figure 10b montre que le vin 5 vieillira plus vite que le vin 4, d'une part, et que le vin 1 (un chef-d'oeuvre) peut être dégusté dès à présent ou d'ici plusieurs années.

En bref, le procédé de l'invention

(1°) est particulièrement adapté au suivi *in vitro* de l'état de santé de l'homme, des animaux et des plantes, en ce qui concerne leur nutrition (et/ou au suivi *in vitro* de l'apport nutritionnel qui leur est fourni), à la gestion de leurs stress et au ralentissement de leur vieillissement, d'une part, et au suivi *in vitro* du potentiel génétique des animaux et des plantes, dans le cadre de leur sélection, d'autre part, et
(2°) met en évidence que les substances antiradicalaires sont essentielles pour prévenir l'encéphalite spongiforme (BSE, OSE ou CJD) et les maladies autoimmunes (notamment la maladie d'Alzheimer, la maladie de Parkinson et les allergies) qui induisent un dérèglement du système de régulation des défenses antiradicalaires globales.

Ainsi, le procédé de l'invention est particulièrement utile pour apprécier, sur le plan antiradicalaire, l'intérêt

nutritif ou la valeur nutritionnelle des aliments, des compléments alimentaires ou diététiques ("nutraceutiques") et des boissons, d'une part, et pour contrôler chaque étape du processus industriel d'élaboration desdits aliments, compléments alimentaires et boissons en vue de préserver la santé de l'homme, de l'animal ou de la plante et de respecter environnement.

On recommande donc selon l'invention, d'une part, l'utilisation du présent procédé de détermination *in vitro* du potentiel de défense antiradicalaire global, ladite utilisation étant caractérisée en ce qu'elle comprend la mise en oeuvre dudit procédé pour le suivi *in vitro* de la croissance des animaux et des végétaux et/ou le suivi *in vitro* de l'apport nutritionnel qui leur est fourni, et d'autre part, l'utilisation dudit procédé de détermination *in vitro* du potentiel de défense antiradicalaire global, ladite utilisation étant caractérisée en ce qu'elle comprend la mise en oeuvre *in vitro* dudit procédé chez l'homme pour le suivi de l'état de santé, la gestion du stress et/ou le suivi du vieillissement.

## Revendications

1. Procédé de détermination *in vitro* du potentiel de défense antiradicalaire global d'un organisme vivant ou d'un agent physique, chimique ou biologique, ledit procédé, qui comporte l'utilisation de radicaux libres en tant que moyen induisant la lyse d'un matériau cellulaire, puis l'évaluation de ladite lyse cellulaire, et selon lequel

    (1°) on met en contact, dans un milieu biologique liquide, un générateur de radicaux libres et un matériau cellulaire qui comporte un colorant libérable lors de la lyse par les radicaux libres, et a été préalablement mis en contact avec un agent chimique ou physique ;
    (2°) on induit la libération des radicaux libres à partir dudit générateur de radicaux libres ; et,
    (3°) on évalue la lyse du matériau cellulaire par une mesure de densité optique, par rapport à un échantillon témoin contenant ledit matériau cellulaire qui n'a pas été mis en contact avec ledit agent chimique ou physique ;

    étant **caractérisé en ce qu'**il comprend l'hydrolyse d'un matériau cellulaire avant ou pendant la libération, dans le milieu réactionnel résultant, de radicaux libres à partir d'un générateur de radicaux libres, ledit matériau cellulaire étant des hématies ou du sang total.

2. Procédé suivant la revendication 1, pour la détermination *in vitro* du potentiel de défense antiradicalaire global d'un organisme vivant ou d'un agent physique, chimique ou biologique, ledit procédé, qui comporte l'utilisation de radicaux libres en tant que moyen induisant la lyse cellulaire, étant **caractérisé en ce qu'**il comprend les étapes suivantes :

    ($\alpha$) hydrolyse d'un échantillon

    (i) d'un premier matériau cellulaire d'un organisme vivant, ledit premier matériau cellulaire étant un tissu cellulaire, qui est du sang total, ou des cellules, qui sont des hématies, ou, respectivement,
    (ii) d'un second matériau cellulaire associé à un agent physique, chimique ou biologique, ledit second matériau cellulaire étant un produit de référence qui est un tissu cellulaire, qui est du sang total, ou des cellules, qui sont des hématies,

    ($\beta$) mise en contact pendant ou après hydrolyse dudit échantillon avec un générateur de radicaux libres,
    ($\gamma$) induction de la libération des radicaux libres à partir dudit générateur de radicaux libres, et
    ($\delta$) suivi de la lyse, par mesure optique, du premier ou, respectivement, du second matériau cellulaire, par rapport à un échantillon témoin, pour apprécier le potentiel antiradicalaire global dudit organisme vivant ou, respectivement, dudit agent physique, chimique ou biologique à tester.

3. Procédé suivant la revendication 2, **caractérisé en ce que** l'on détermine à l'étape ($\delta$) la valeur T½ qui correspond à la lyse de 50 % du premier matériau cellulaire ou du second matériau cellulaire et qui exprime le potentiel antiradicalaire global dudit premier matériau cellulaire ou dudit matériau cellulaire associé audit agent physique, chimique ou biologique.

4. Procédé suivant la revendication 2 ou 3, **caractérisé en ce que** ledit premier matériau cellulaire est du sang total.

5. Procédé suivant la revendication 2 ou 3, **caractérisé en ce que** ledit second matériau cellulaire est du sang total.

6. Procédé suivant la revendication 2 ou 3, **caractérisé en ce que** ledit agent biologique est le plasma d'un sujet humain ou d'un animal à sang chaud.

**7.** Procédé suivant la revendication 2, **caractérisé en ce que** l'hydrolyse du stade (α) est (i) une hydrolyse enzymatique, (ii) une hydrolyse acide ou alcaline, ou (iii) un clivage au moyen d'un agent physique, notamment un rayonnement.

**8.** Procédé pour apprécier la résistance antiradicalaire d'un agent physique, chimique ou biologique, ledit procédé étant **caractérisé en ce qu'**il comprend les étapes suivantes :

(α) hydrolyse d'un échantillon
d'un colorant dégradable par les radicaux libres et qui est associé
à un agent physique, chimique ou biologique,
(β) mise en contact, pendant ou après ladite hydrolyse, dudit échantillon avec un générateur de radicaux libres,
(γ) induction de la libération des radicaux libres à partir dudit générateur de radicaux libres, et
(δ) suivi de la lyse, par mesure optique, dudit colorant par rapport à un échantillon témoin, pour apprécier le potentiel antiradicalaire global dudit échantillon contenant ledit agent physique, chimique ou biologique à tester.

**9.** Procédé suivant la revendication 8, **caractérisé en ce que** ledit colorant est le bleu de méthylène ou le colorant rouge extrait d'oligo-polyanthocyanidine.

**10.** Utilisation du procédé selon l'une quelconque des revendications 1 à 9, **caractérisée en ce qu'**elle comprend la mise en oeuvre dudit procédé pour le suivi *in vitro* de la croissance des animaux et des végétaux et/ou le suivi *in vitro* de l'apport nutritionnel qui leur est fourni.

**11.** Utilisation du procédé selon l'une quelconque des revendications 1 à 9, **caractérisée en ce qu'**elle comprend la mise en oeuvre *in vitro* dudit procédé chez l'homme pour le suivi de l'état de santé, la gestion du stress et/ou le suivi du vieillissement.

**12.** Utilisation suivant la revendication 10 ou 11, **caractérisée en ce que** la substance antiradicalaire est l'acide férulique sulfaté ou l'un des osides dudit acide férulique, notamment le glucuronide.

**13.** Utilisation suivant la revendication 10, pour le suivi du vieillissement du vin.

## Claims

**1.** A method for the *in vitro* determination of the overall antiradical defence potential of a living organism or of a physical, chemical or biological agent, said method, which involves the use of free radicals as a means for inducing cell lysis followed by evaluation of said cell lysis, and comprising the following steps:

(1) a free radical generator is brought into contact, in an appropriate liquid biological medium, with a cellular material which comprises a colorant which can be released on lysis by free radicals, and has first been brought into contact with a physical or chemical agent;
(2) the release of free radicals from said free radical generator is induced; and,
(3) lysis of the cellular material is evaluated by measuring optical density relative to a control sample containing said cellular material which has not been brought into contact with said physical or chemical agent; being **characterised in that** it comprises hydrolysis of a cellular material before or during the release, in the resultant reaction medium, of free radicals from a free radical generator, said cellular material being erythrocytes or whole blood.

**2.** A method according to claim 1 for the *in vitro* determination of the overall antiradical defence potential of a living organism or of a physical, chemical or biological agent, said method, which involves the use of free radicals as a means for inducing cell lysis, being **characterised in that** it comprises the following steps:

(α) hydrolysis of a sample

(i) of a first cellular material from a living organism, said first cellular material being a cellular tissue, which is whole blood, cells which are erythrocytes, or, respectively,
(ii) of a second cellular material associated with a physical, chemical or biological agent, said second cellular material being a reference product which is a cellular tissue, which is whole blood, cells which are erythrocytes,

(β) during or after said hydrolysis, bringing said sample into contact with a free radical generator,
(γ) inducing release of the free radicals from said free radical generator, and
(δ) monitoring lysis, by optical measurement, of the first or, respectively, second cellular material, relative to a control sample, in order to assess the overall antiradical potential of said living organism or, respectively, of said physical, chemical or biological agent to be tested.

3. A method according to claim 2, **characterised in that**, in step (δ), the T½ value is determined which corresponds to the lysis of 50% of the first cellular material or the second cellular material and which expresses the overall antiradical potential of said first cellular material or of said cellular material associated with said physical, chemical or biological agent.

4. A method according to claim 2 or claim 3, **characterised in that** said first cellular material is whole blood.

5. A method according to claim 2 or claim 3, **characterised in that** said second cellular material is whole blood.

6. A method according to claim 2 or claim 3, **characterised in that** said biological agent is plasma from a human subject or from a warm-blooded animal.

7. A method according to claim 2, **characterised in that** the hydrolysis of stage (α) is (i) enzymatic hydrolysis, (ii) acid or alkaline hydrolysis, or (iii) cleaving by means of a physical agent, in particular radiation.

8. A method for assessing the antiradical resistance of a physical, chemical or biological agent, said method being **characterised in that** it comprises the following steps:

(α) hydrolysis of a sample
of a colorant which is degradable by free radicals and which is associated with a physical, chemical or biological agent,
(β) during or after said hydrolysis, bringing said sample into contact with a free radical generator,
(γ) inducing release of the free radicals from said free radical generator, and
(δ) monitoring the lysis, by optical measurement, of said colorant relative to a control sample, in order to assess the overall antiradical potential of said sample containing said physical, chemical or biological agent to be tested.

9. A method according to claim 8, **characterised in that** said colorant is methylene blue or the red colorant extracted from oligoanthocyanidin.

10. Use of the method according to any one of claims 1 to 9, **characterised in that** it comprises the performance of said method for *in vitro* monitoring of the growth of animals and plants and/or *in vitro* monitoring of the nutritional input provided to them.

11. Use of the method according to any one of claims 1 to 9, **characterised in that** it comprises the *in vitro* performance of said method in humans for monitoring the state of health, managing stress and/or monitoring ageing.

12. Use according to claim 10 or claim 11, **characterised in that** the antiradical substance is sulfated ferulic acid or one of the osides of said ferulic acid, in particular glucuronide.

13. Use according to claim 10 for monitoring the ageing of wine.

**Patentansprüche**

1. Verfahren zur in vitro Bestimmung des globalen antiradikalischen Abwehrpotentials eines lebenden Organismus oder eines physikalischen, chemischen oder biologischen Mittels, wobei das Verfahren die Verwendung von freien, die Lyse eines zellulären Materials induzierenden Radikalen als Mittel umfasst und dann die Evaluierung der zellulären Lyse, und gemäß welchem

(1°) ein Erzeuger freier Radikale und ein zelluläres Material, das einen Farbstoff umfasst, der bei der Lyse durch die freien Radikale frei wird und zuvor in Kontakt mit einem chemischen oder physikalischen Mittel gebracht worden war, in einem flüssigen, biologischen Milieu miteinander in Kontakt gebracht wird;

(2°) das Freisetzen der freien Radikale durch einen Erzeuger freier Radikale induziert wird; und
(3°) die Lyse des zellulären Materials durch eine Messung der optischen Dichte im Vergleich zu einer markierten Probe, die das zelluläre Material, das nicht in Kontakt mit dem chemischen oder physikalischen Mittel gebracht worden war, enthält, evaluiert wird;

**dadurch gekennzeichnet, dass** das Verfahren die Hydrolyse eines zellulären Materials vor oder während des Freisetzens der freien Radikale durch einen Erzeuger freier Radikale in dem resultierenden, reagierenden Milieu umfasst, wobei das zelluläre Material aus Erythrozyten oder aus Vollblut besteht.

**2.** Verfahren gemäß Anspruch 1 zur in vitro Bestimmung des globalen antiradikalischen Abwehrpotentials eines lebenden Organismus oder eines physikalischen, chemischen oder biologischen Mittels, wobei das Verfahren die Verwendung freier, die zelluläre Lyse induzierenden Radikale als Mittel umfasst, **dadurch gekennzeichnet, dass** es die folgenden Schritte umfasst:

(α) Hydrolyse einer Probe

(i) eines ersten zellulären Materials eines lebenden Organismus, wobei das erste zelluläre Material ein zelluläres Gewebe ist, das aus Vollblut oder aus Zellen, die Erythrozyten sind, besteht, oder, jeweils
(ii) eines zweiten, mit einem physikalischen, chemischen oder biologischen Mittel verbundenen, zellulären Materials, wobei das zweite zelluläre Material ein Referenzprodukt ist, das ein zelluläres Gewebe ist, das aus Vollblut oder aus Zellen, die Erythrozyten sind, besteht,

(β) in Kontakt bringen der Probe mit einem Erzeuger freier Radikale während oder nach Hydrolyse,
(γ) Induktion des Freisetzens der freien Radikale durch den Erzeuger freier Radikale, und
(δ) Überwachung der Lyse des ersten beziehungsweise des zweiten zellulären Materials im Vergleich zu einer markierten Probe durch optische Messung, um das globale antiradikalische Abwehrpotential des lebenden Organismus beziehungsweise des zu testenden physikalischen, chemischen oder biologischen Mittels zu bewerten.

**3.** Verfahren gemäß Anspruch 2, **dadurch gekennzeichnet, dass** in Schritt (δ) der Wert T1/2 bestimmt wird, der der Lyse von 50 % des ersten zellulären Materials oder des zweiten zellulären Materials entspricht und der das globale antiradikalische Potential des ersten zellulären Materials oder des mit dem physikalischen, chemischen oder biologischen Mittel verbundenen zellulären Materials ausdrückt.

**4.** Verfahren gemäß Anspruch 2 oder 3, **dadurch gekennzeichnet, dass** das erste zelluläre Material aus Vollblut ist.

**5.** Verfahren gemäß Anspruch 2 oder3, **dadurch gekennzeichnet, dass** das zweite zelluläre Material aus Vollblut ist.

**6.** Verfahren gemäß Anspruch 2 oder 3, **dadurch gekennzeichnet, dass** das biologische Mittel Plasma eines menschlichen Subjektes oder eines warmblütigen Tieres ist.

**7.** Verfahren gemäß Anspruch 2, **dadurch gekennzeichnet, dass** die Hydrolyse der Stufe (α) eine enzymatische Hydrolyse (i), eine saure oder alkalische Hydrolyse (ii), oder eine Spaltung mit Hilfe eines physikalischen Mittels (iii), insbesondere einer Strahlung ist.

**8.** Verfahren zur Bewertung der antiradikalischen Widerstandsfähigkeit eines physikalischen, chemischen oder biologischen Mittels, wobei das Verfahren **dadurch gekennzeichnet ist, dass** es die folgenden Schritte umfasst:

(α) Hydrolyse einer Probe
eines Farbstoffs, der durch die freien Radikale abbaubar ist und der mit einem physikalischen, chemischen oder biologischen Mittel verbunden ist,
(β) in Kontakt bringen der Probe mit einem Erzeuger freier Radikale während oder nach der Hydrolyse,
(γ) Induktion des Freisetzens der freien Radikale durch den Erzeuger freier Radikale, und
(δ) Überwachung der Lyse des Farbstoffs im Vergleich zu einer markierten Probe durch optische Messung, um das globale antiradikalische Potential der Probe, die das zu testende physikalische, chemische oder biologische Mittel enthält, zu bewerten.

**9.** Verfahren gemäß Anspruch 8, **dadurch gekennzeichnet, dass** der Farbstoff Methylenblau oder der aus Oligopolyanthocyanidin extrahierte, rote Farbstoff ist.

10. Verwendung des Verfahrens gemäß einem der Ansprüche 1 bis 9, **dadurch gekennzeichnet, dass** sie die Durchführung des Verfahrens für die in vitro Überwachung des Wachstums der Tiere und der Pflanzen und/oder die in vitro Überwachung der Nährstoffzufuhr, die ihnen geliefert wird, umfasst.

11. Verwendung des Verfahrens gemäß einem der Ansprüche 1 bis 9, **dadurch gekennzeichnet, dass** sie die in vitro Durchführung des Verfahrens beim Menschen zur Überwachung des Gesundheitszustands, der Bewältigung von Stress und/oder zur Überwachung des Alterns umfasst.

12. Verwendung gemäß Anspruch 10 oder 11, **dadurch gekennzeichnet, dass** die antiradikalische Substanz sulfatierte Ferulasäure oder eines der Oside der Ferulasäure, insbesondere das Glucuronid ist.

13. Verwendung gemäß Anspruch 10 zur Überwachung der Alterung von Wein.

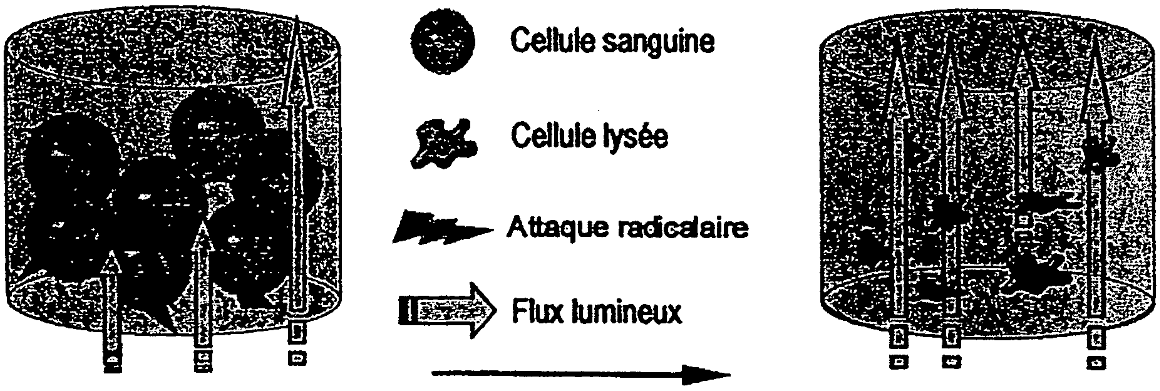
Cellule sanguine
Cellule lysée
Attaque radicalaire
Flux lumineux

Fig. 1

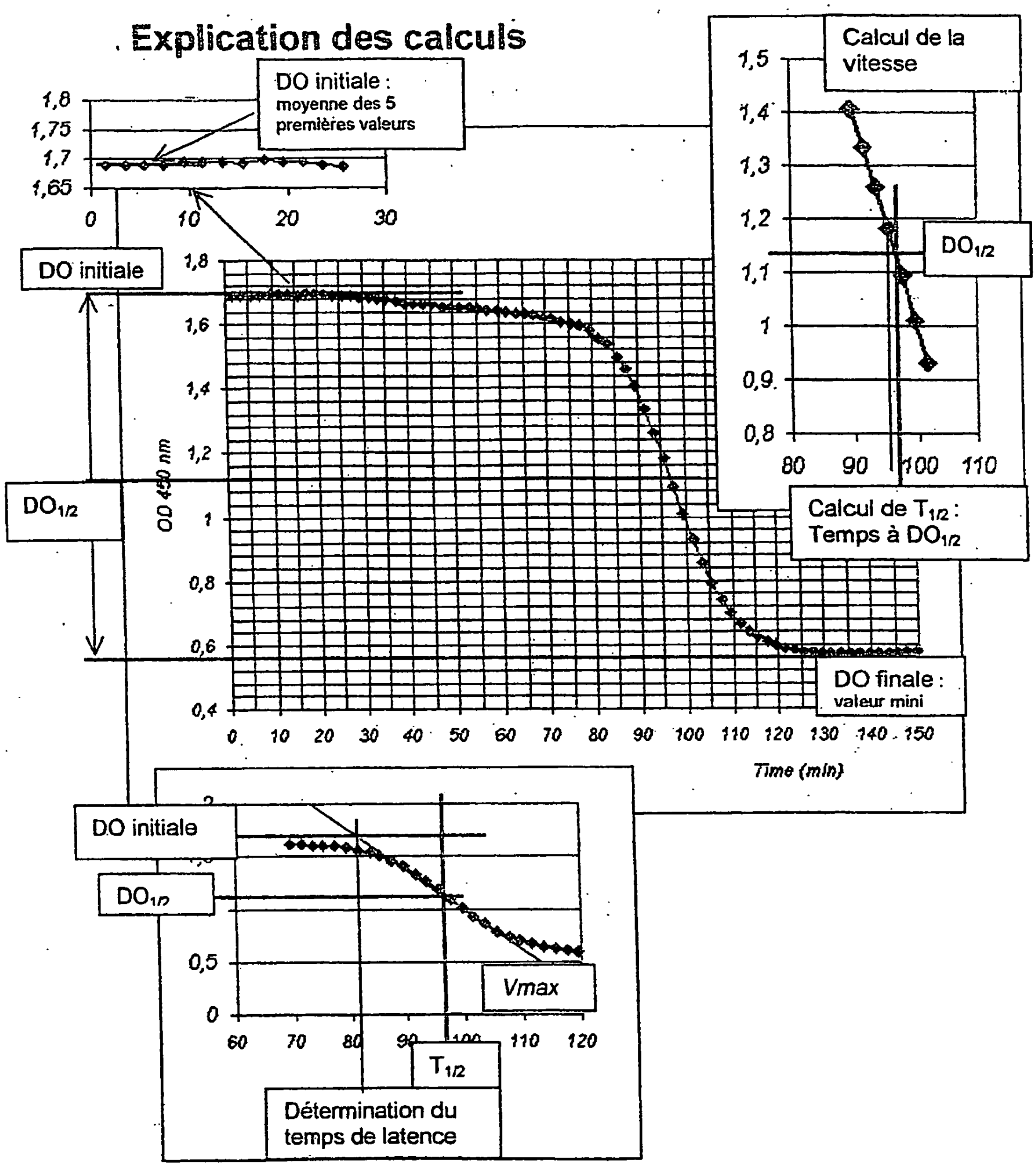
Explication des calculs
DO initiale : moyenne des 5 premières valeurs
1,8
1,75
1,7
1,65
0
10
20
30
DO initiale
DO1/2
OD 450 nm
1,8
1,6
1,4
1,2
1
0,8
0,6
0,4
0 10 20 30 40 50 60 70 80 90 100 110 120 130 140 150
Time (min)
DO finale : valeur mini
Calcul de la vitesse
1,5
1,4
1,3
1,2
1,1
1
0,9
0,8
80 90 100 110
DO1/2
Calcul de T1/2 : Temps à DO1/2
DO initiale
DO1/2
0,5
0
60 70 80 90 100 110 120
Vmax
T1/2
Détermination du temps de latence

Fig. 2

**Résistance d'un sang témoin à l'attaque radicalaire en présence d'une forme aglycone de flavonoïde: Quercetine et de sa forme glycone: Isoquercitrine**

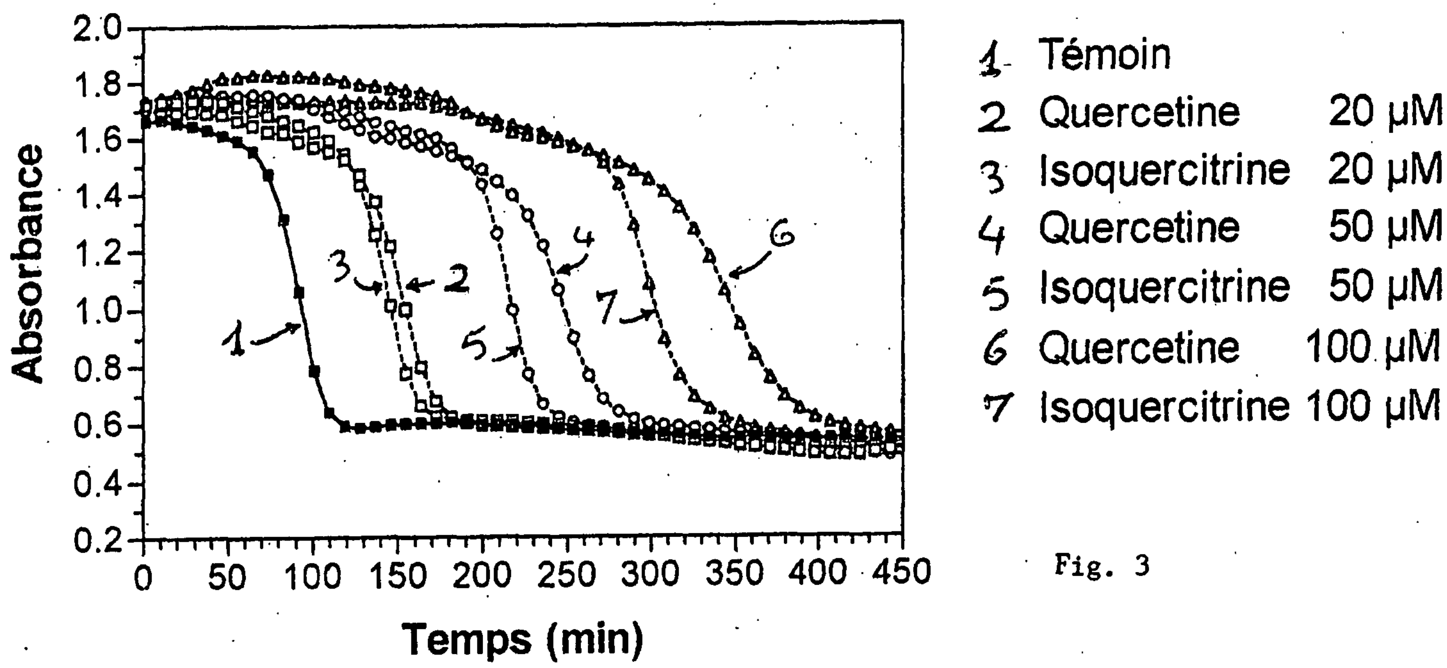


Fig. 3

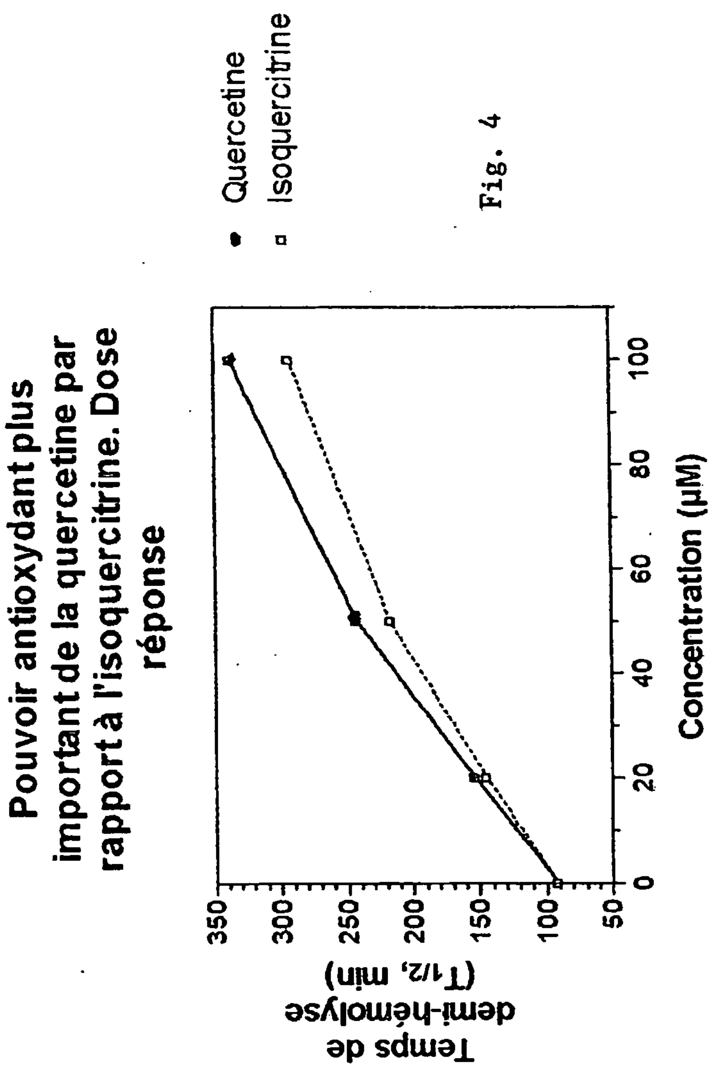
Pouvoir antioxydant plus important de la quercetine par rapport à l'isoquercitrine. Dose réponse
Quercetine
Isoquercitrine
Temps de demi-hémolyse (T1/2, min)
350
300
250
200
150
100
50
0
20
40
60
80
100
Concentration (µM)

Fig. 4

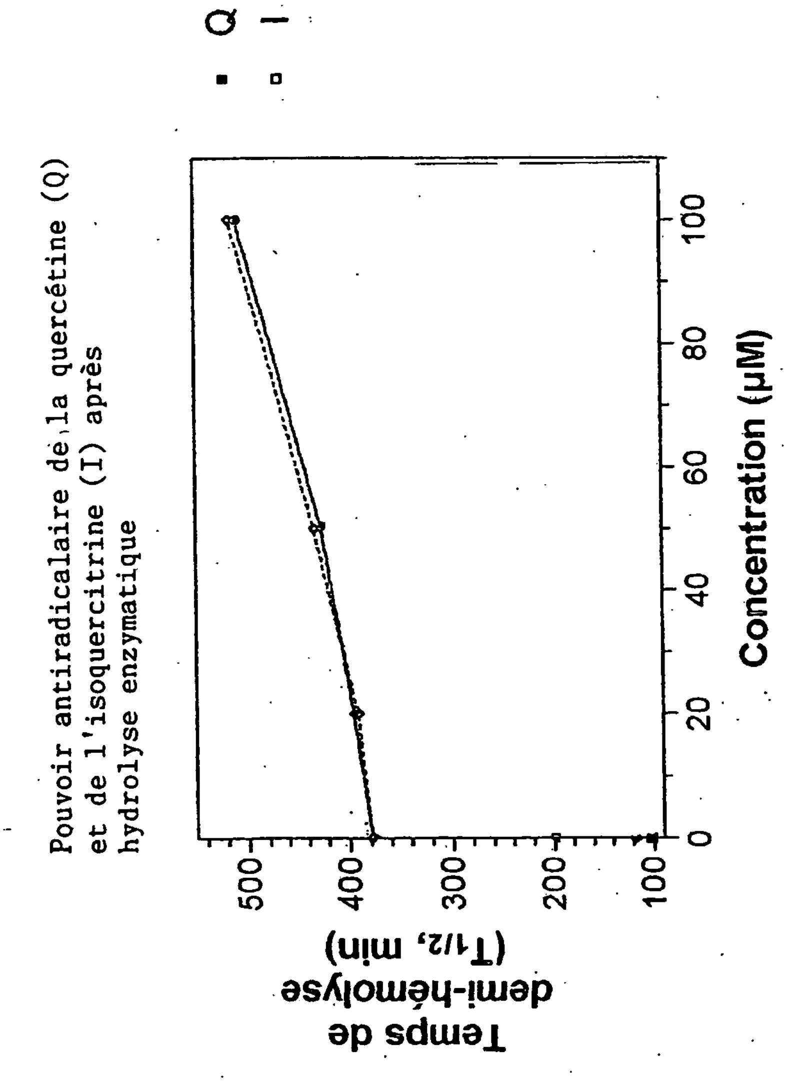

Pouvoir antiradicalaire de la quercétine (Q)
et de l'isoquercitrine (I) après
hydrolyse enzymatique
Temps de
demi-hémolyse
(T1/2, min)
500
400
300
200
100
0
20
40
60
80
100
Concentration (µM)
Q
I


Fig. 5

# Influence d'un traitement de la vigne sur l'efficacité antiradicalaire d'échantillons de raisin

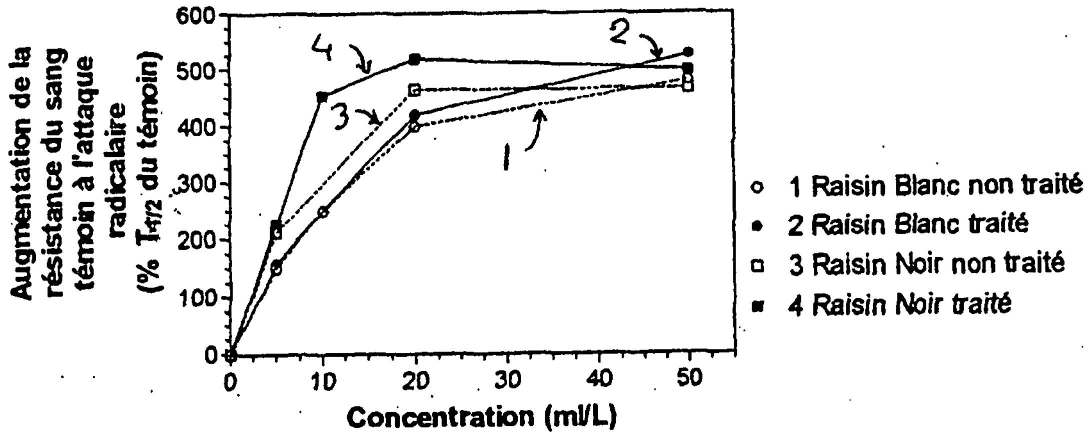


Fig. 6

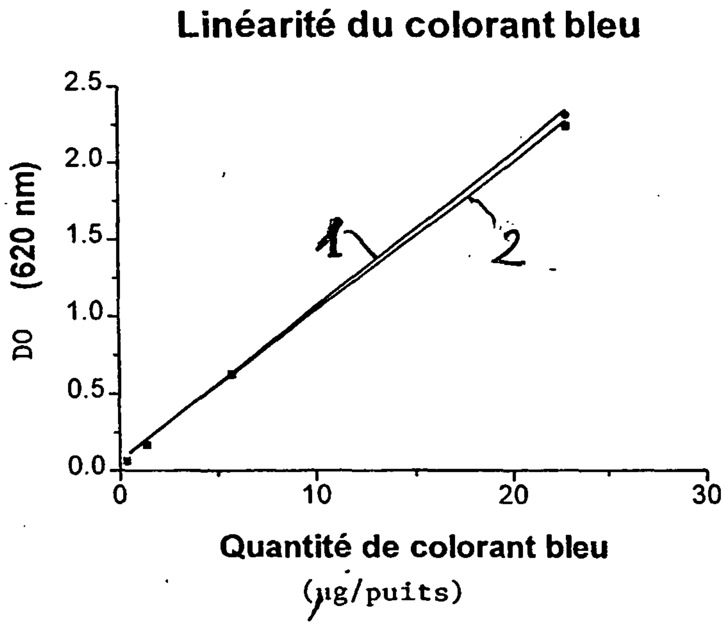
Linéarité du colorant bleu
DO (620 nm)
2.5
2.0
1.5
1.0
0.5
0.0
0
10
20
30
1
2
Quantité de colorant bleu
(µg/puits)

Fig. 7a

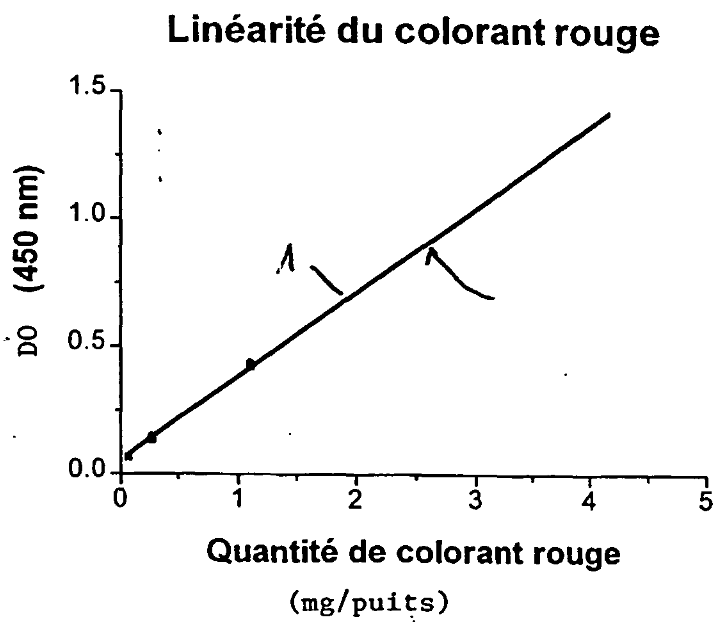
Linéarité du colorant rouge
DO (450 nm)
1.5
1.0
0.5
0.0
0
1
2
3
4
5
1
Quantité de colorant rouge
(mg/puits)

Fig 7b

**Effet du générateur de radicaux libres sur le colorant bleu au cours du temps**
**(courbe moyenne obtenue à partir de 6 puits identiques)**

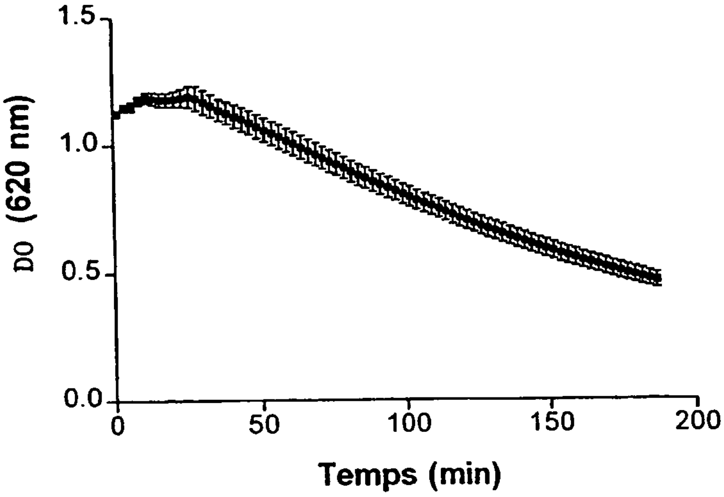


Fig. 7c

**Effet du générateur de radicaux libres sur le colorant rouge au cours du temps**
**(courbe moyenne obtenue à partir de 6 puits identiques)**

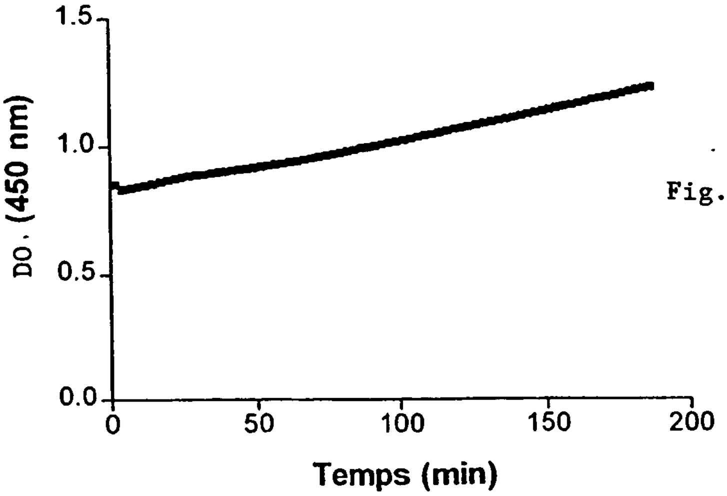


Fig. 7d

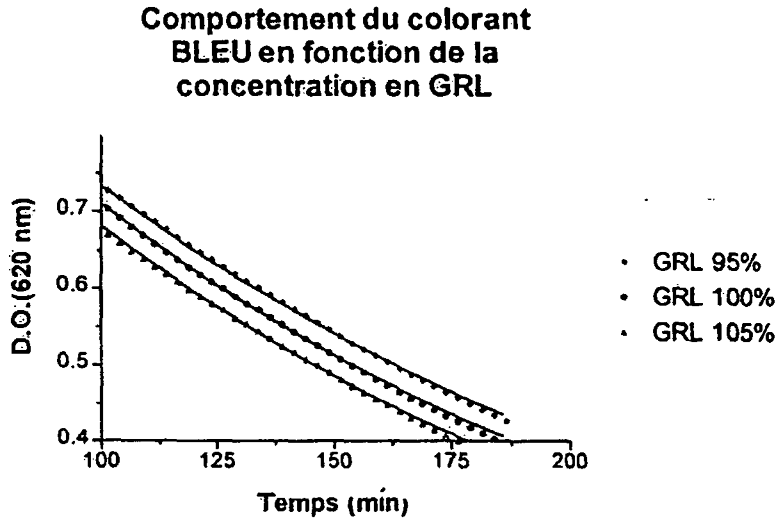
Comportement du colorant
BLEU en fonction de la
concentration en GRL
D.O.(620 nm)
0.7
0.6
0.5
0.4
100
125
150
175
200
Temps (min)
GRL 95%
GRL 100%
GRL 105%

Fig 7e

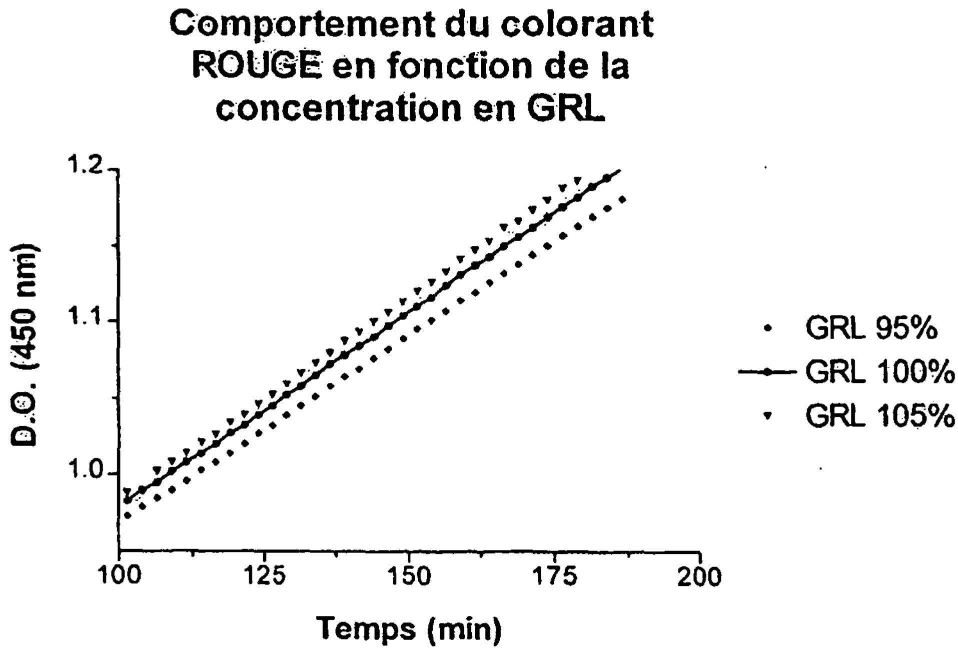
Comportement du colorant
ROUGE en fonction de la
concentration en GRL
D.O. (450 nm)
1.2
1.1
1.0
100
125
150
175
200
Temps (min)
GRL 95%
GRL 100%
GRL 105%

Fig 7f

**Résistance antiradicalaire du sang entier de 4 souches de Gallinacées**

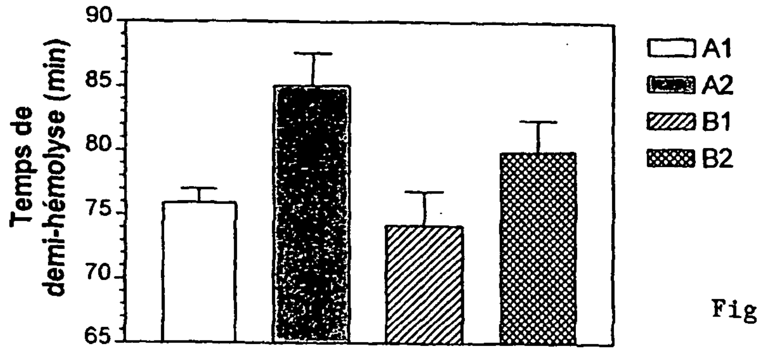


Fig. 8a

**Résistance antiradicalaire des hématies de de 4 souches de Gallinacées**

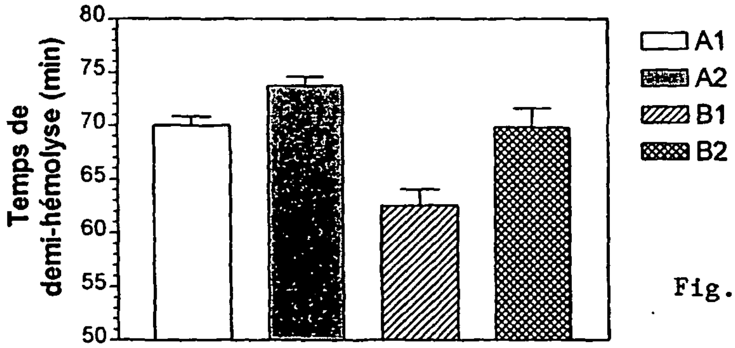


Fig. 8b

**Efficacité antiradicalaire du plasma de de 4 souches de Gallinacées**

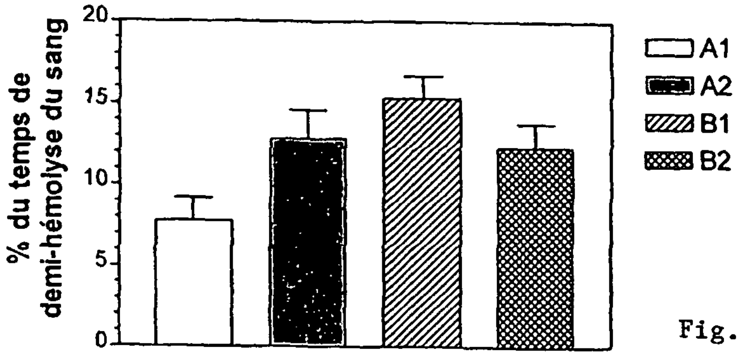


Fig. 8c

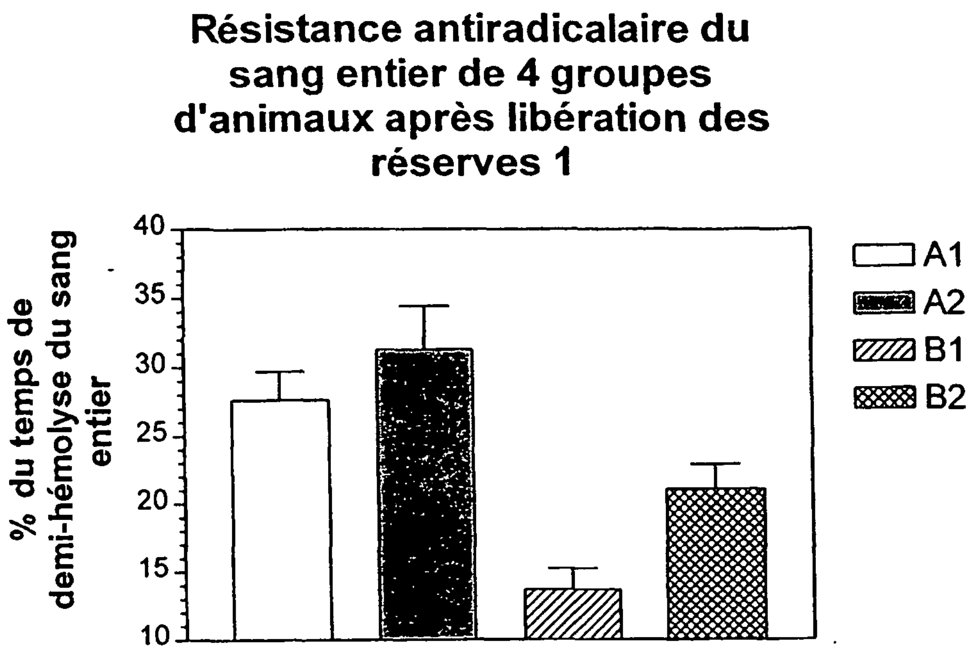
Résistance antiradicalaire du sang entier de 4 groupes d'animaux après libération des réserves 1
% du temps de demi-hémolyse du sang entier
40
35
30
25
20
15
10
A1
A2
B1
B2

Fig. 8d

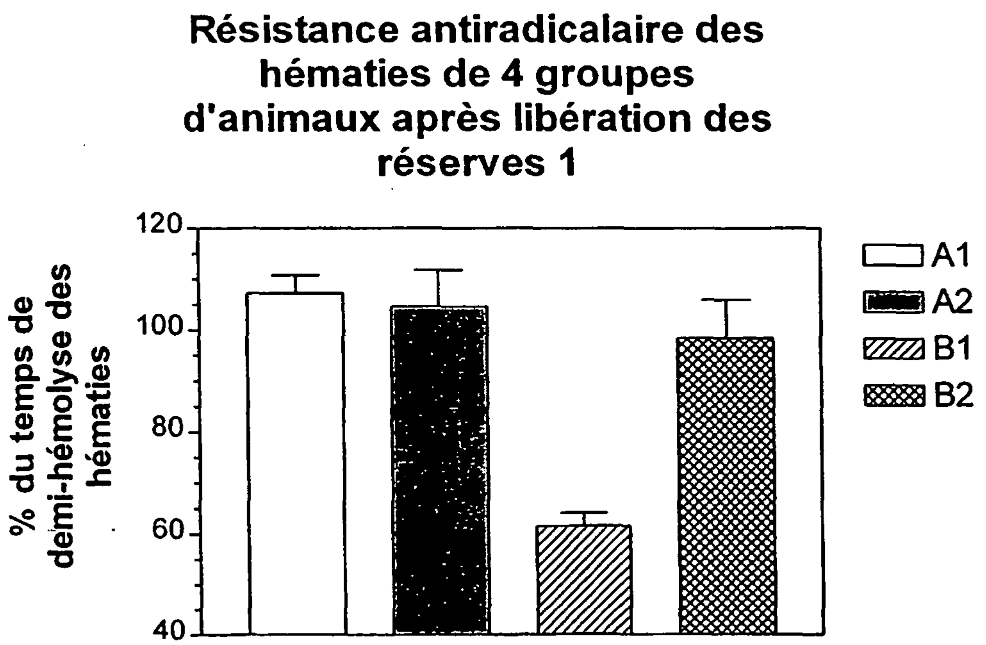
Résistance antiradicalaire des hématies de 4 groupes d'animaux après libération des réserves 1
% du temps de demi-hémolyse des hématies
120
100
80
60
40
A1
A2
B1
B2

Fig. 8e

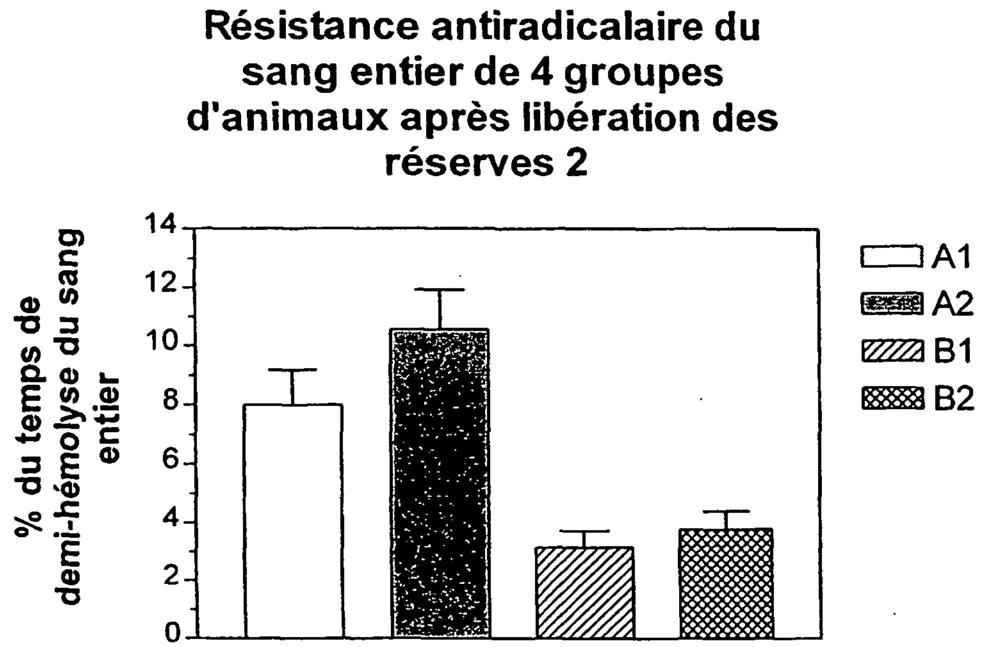
Résistance antiradicalaire du sang entier de 4 groupes d'animaux après libération des réserves 2
% du temps de demi-hémolyse du sang entier
14
12
10
8
6
4
2
0
A1
A2
B1
B2

Fig. 8f

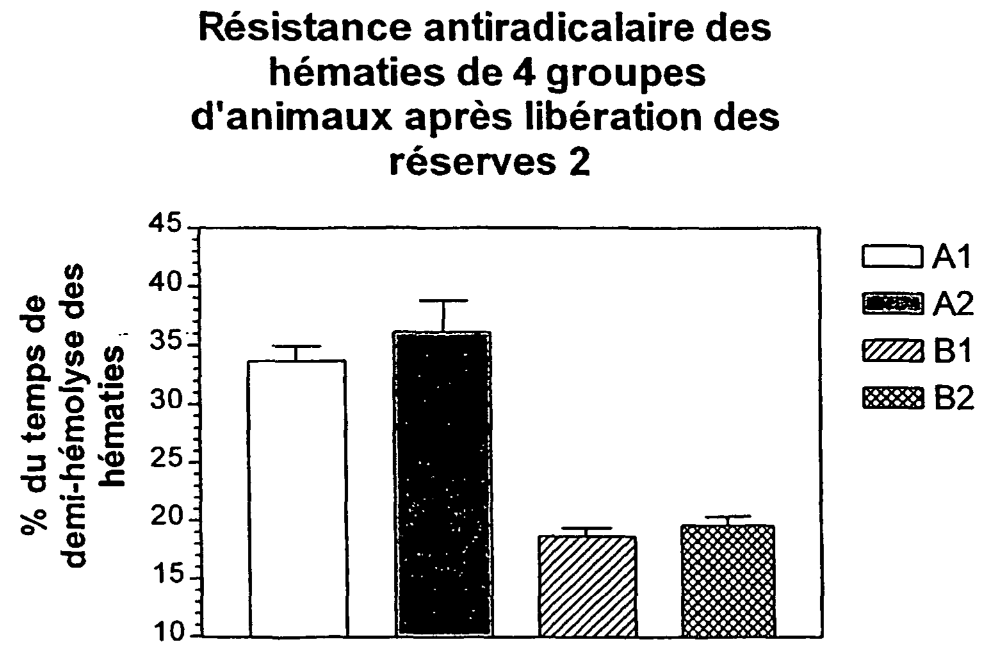
Résistance antiradicalaire des hématies de 4 groupes d'animaux après libération des réserves 2
% du temps de demi-hémolyse des hématies
45
40
35
30
25
20
15
10
A1
A2
B1
B2

Fig. 8g

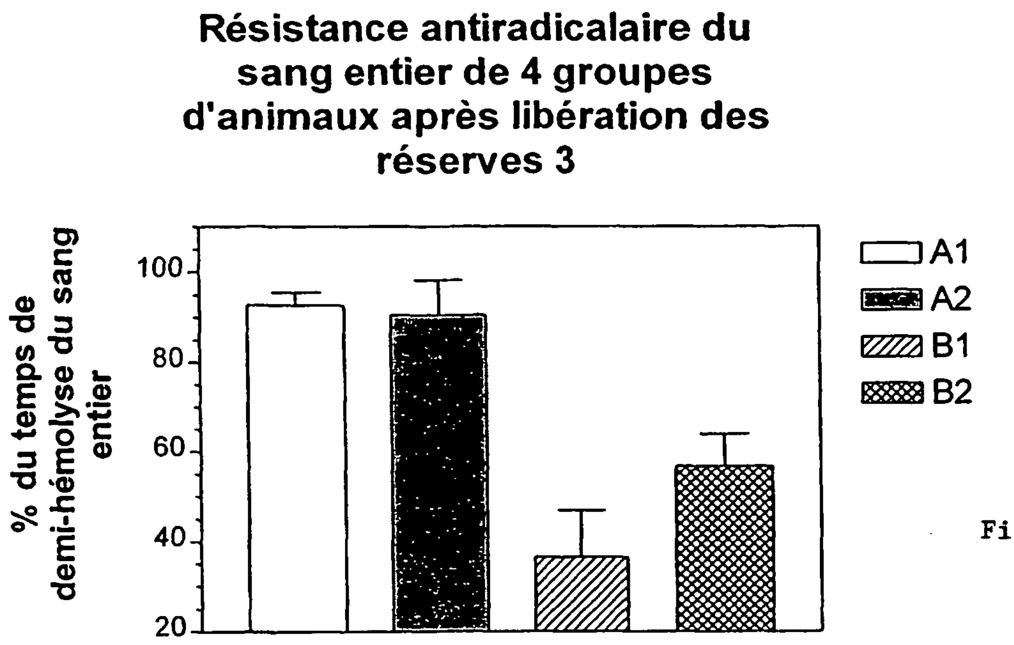
Résistance antiradicalaire du sang entier de 4 groupes d'animaux après libération des réserves 3
% du temps de demi-hémolyse du sang entier
100
80
60
40
20
A1
A2
B1
B2

Fig. 8h

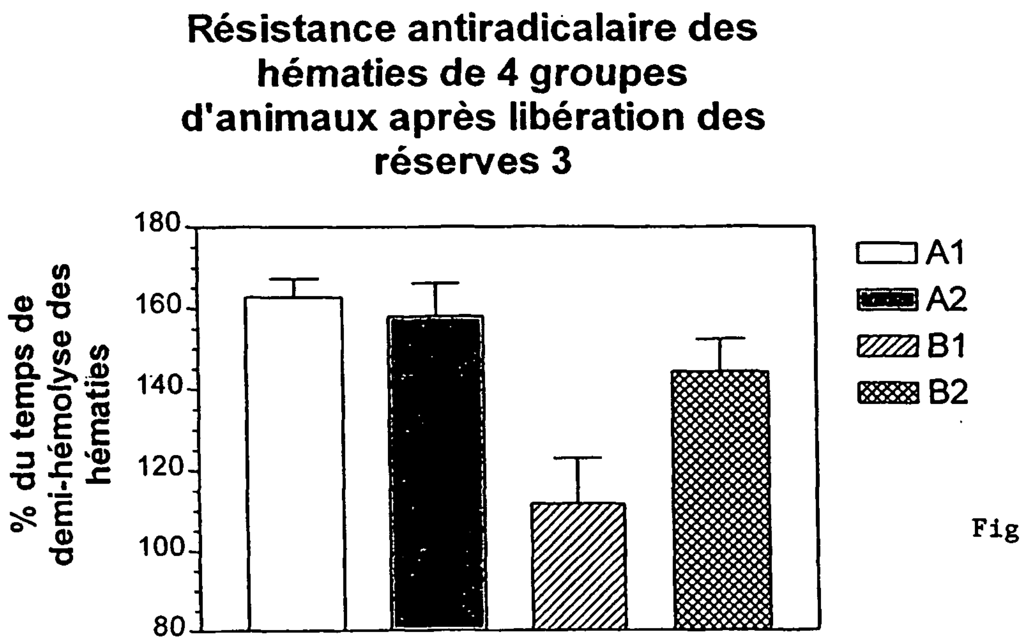
Résistance antiradicalaire des hématies de 4 groupes d'animaux après libération des réserves 3
% du temps de demi-hémolyse des hématies
180
160
140
120
100
80
A1
A2
B1
B2

Fig. 8i

Test KRL (T1/2) et réserves R1, R2 et R3 (%) sur hématies

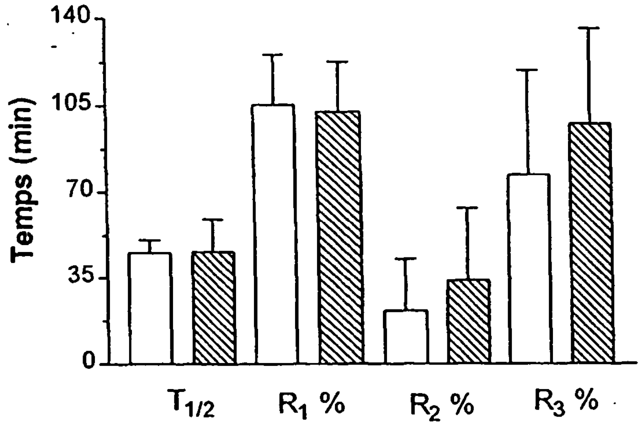


Hématies (ou RBC)
Comparaison rats témoins et diabétiques (Streptozotocyne)

Fig. 9a

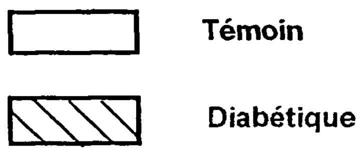


Test KRL (T1/2) et réserves R1, R2 et R3 (%) sur sang total

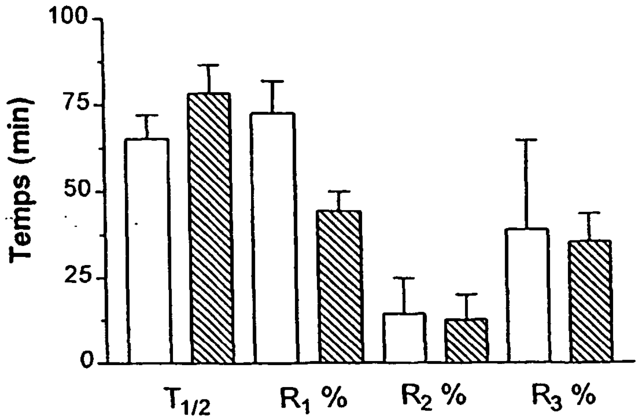


Sang Total
Comparaison rats témoins et diabétiques (Streptozotocyne)

Fig. 9b

**Test KRL (T1/2) et réserves R1, R2 et R3 (%) sur hématies**

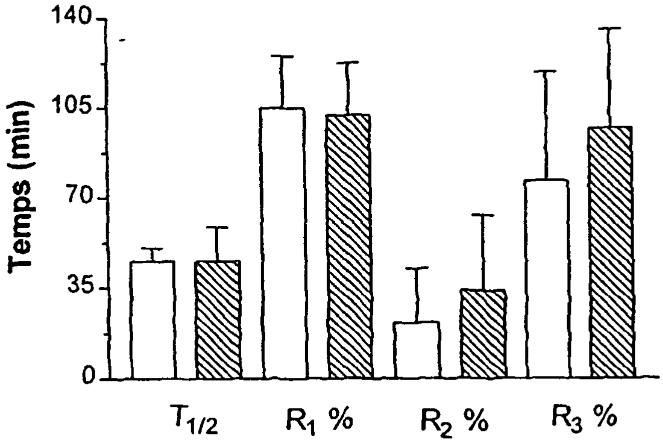


**Hématies (ou RBC)**
**Comparaison rats témoins et diabétiques (Streptozotocyne)**

Fig. 9c

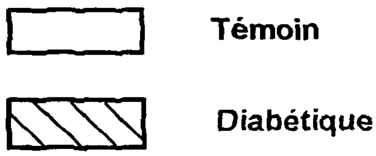


**Test KRL (T1/2) et réserves R1, R2 et R3 (%) sur sang total**

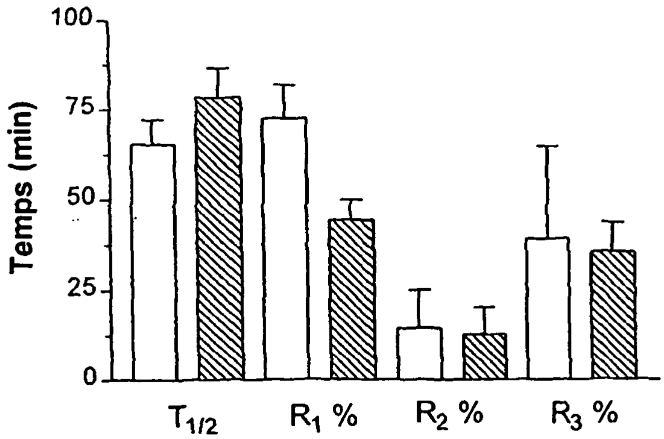


**Sang Total**
**Comparaison rats témoins et diabétiques (Streptozotocyne)**

Fig. 9d

**Vieillissement accéléré de différents vins**

**conservé dans sa bouteille d'origine à température ambiante à l'abri de la lumière et ouverte à chaque prélèvement à une concentration de 2ml/L dans le milieu réactionnel**

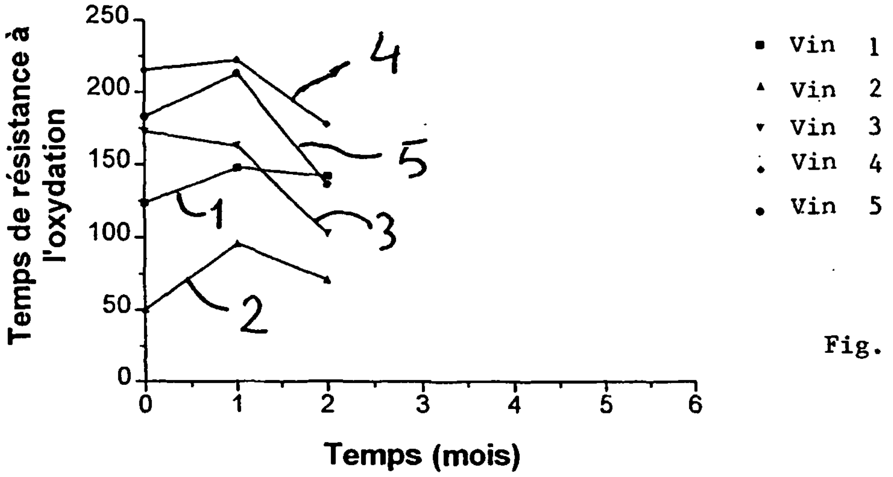


Fig. 10a

**Comparaison des réserves anti-radicalaires de différents vins pour le suivi de leur courbe de vieillissement**

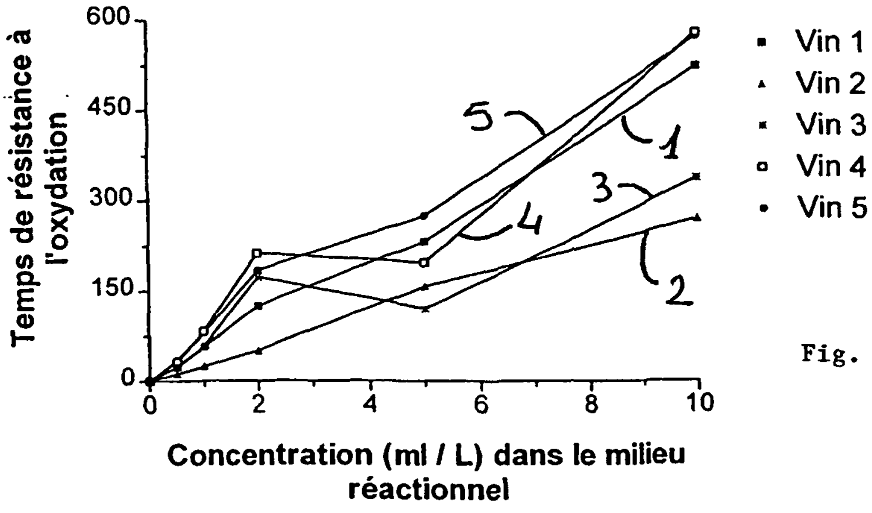


Fig. 10b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0418335 B


### Littérature non-brevet citée dans la description


- **JEAN-FRANÇOIS LESGARDS et al.** *Environmental Health Perspectives,* Mai 2002, vol. 110 (5), 1-9
- **JAN STAGSTED et al.** *Free Radical Research,* 2002, vol. 36 (7), 779-789
- **STUART TURNBULL et al.** *Neuroreport,* 2003, vol. 14 (13), 106-126
- **A. BARRET et al.** *J. Virol.,* 2003, vol. 77 (15), 8462-8469
- **SABRINA CAPELLARI et al.** *J. Biol. Chem.,* 1999, vol. 274 (49), 34846-34850